# EUROPEAN PATENT SPECIFICATION

(11) **EP 0 920 449 B1**
(45) Date of publication and mention of the grant of the patent: **23.10.2002**
(21) Application number: 97942832.3
(22) Date of filing: 26.07.1997
(51) Int. Cl.: C07K 14/12, A61K 39/17

(54) **MORBILLIVIRUS-DERIVED PEPTIDES**
PEPTIDE ABGELEITET AUS DEM MORBILLIVIRUS
PEPTIDES DU VIRUS MORBILLI

(30) Priority: 31.07.1996 EP 96112341
(43) Date of publication of application: 09.06.1999
(73) Proprietor: Muller, Claude P., 7338 Heisdorf (LU)
(72) Inventor: Muller, Claude P., 7338 Heisdorf (LU)
(74) Representative: VOSSIUS & PARTNER
(86) International application number: EP9704070
(87) International publication number: WO98005682

(56) References cited:
- EP-A- 0 540 135
- OBEID E.O. ET AL.: "Analysis of th antigenic profile of measles virus haemagglutinin in mice and humans using overlypping synthesis peptides" VIRUS RESEARCH, vol. 32, 1994, pages 69-84, XP002062540
- FOURNIER PH. ET AL.: "Antibodies to a new linear site at the topographical or functional interface between the haemagglutin and fusion proteins protect against measles encephalitis" J. GENERAL VIROLOGY, vol. 78, 6 June 1997, pages 1295-1302, XP002062541
- ZIEGLER D. ET AL.: "Protection against measles virus encephalitis by monoclonal antibodies binding to a cystein loop domain of the H protein mimicked by peptides which are not recognized by maternal antibodies" J. GENERAL VIROLOGY, vol. 77, 10 October 1996, pages 2479-2489, XP002062542
- ROTA J.S. ET AL.: "Comparison of sequences of the H, F, and N coding genes of measles virus vaccine strains" VIRUS RESEARCH, vol. 31, 1994, pages 317-330, XP002062543

## Description

### Technical Field

The present invention relates to vaccines comprising peptides having an amino acid sequence corresponding to or imitating at least one sequential antigenic determinant of the hemagglutinin protein of a morbillivirus, wherein said antigenic determinant is the binding site of a neutralizing and protective antibody of a morbillivirus to certain sequential antigenic determinants and to peptides comprising such antigenic determinants.

### Background Art

Morbillivirus such as, for example the measles, virus belong to Paramyxoviridae of RNA virus.

Measles virus was first isolated in cell culture in 1954 (Enders, J.F. et al, Proc. Soc. Exp. Biol. Med., 86, 227-286, 1954) from David Edmonston. The Edmonston strain of measles virus became the progenitor for many live attenuated measles vaccine strains.

Current live attenuated measles virus vaccines have effectively reduced morbidity and mortality of measles world-wide. However, while the incidence of measles has been reduced to sporadic cases in the northern hemisphere, the lack of resistance to passively transferred maternal antibodies and the thermal lability of the live measles vaccines are among the major impediments towards their comprehensive application in developing countries and to global eradication of measles (see Weis, R., Science 258, 546-547 (1992)).

Efforts to curb the measles resurgence has focused on the role played by measles structural proteins in inducing immunity in a vaccinated mammal. The measles virus, like many members of the paramyxovirus family, contains six major structural proteins: the matrix protein, the hemagglutinin protein, the fusion protein, the large protein, the phosphoprotein and the nucleocapsid protein. Of these, the envelope glycoprotein, hemagglutinin and fusion protein have been shown to be responsible for induction of measles virus neutralizing antibodies (see Varsanyl et al.; J. Gen. Viriol. 65, 365 (1984); Giraudon et al. Virology 144, 46 (1985); and Drillien et al. Proc. Nat'l. Acad. Sci. USA 85, 1252-56 (1988)). Maternal antibodies (Albrecht 1977) and hyperimmune globulin (Janeway 1949) demonstrate the importance of antibodies in protecting against measles virus infection. The hemagglutinin protein is known to be the prime target for neutralizing and protective antibodies (Giraudon & Wild, 1985; Varsanyi et al. 1987).

Competitive binding assays with monoclonal antibodies have been used to identify distinct antigenic sites of the H-protein (Sheshberadaran et al., Virology, 152, 58-65 (1986); Carter et al., J. Gen. Virol, 63, 113-120 (1982); Giraudon et al. Viroloay 144, 46 (1985); ter Meulen et al., J. Gen. Virol., 57, 357-364 (1981). Hu et al., Virology, 192, 351-354 (1993) and Liebert et al., J. Virol., 68, 1486-1493 (1994) have mapped neutralizing epitopes of measles virus hemagglutinin (MV-H) by sequencing escape mutants resistant to neutralizing antibodies. With this approach, amino acids were identified which were important for the integrity of the monoclonal antibodies (mAb) binding sites. In general neutralizing antibodies are mostly directed against conformational epitopes.

In other viruses, linear epitopes have been found to be neutralizing and sometimes protective e.g. in foot and mouth disease (Francis et al. Immunology, 73. 249-254 (1991)) and influenza virus (Shapira et al., Proc. Natl. Acad. Sci. USA, 81 2461-2465 (1984)). Peptides mimicking epitopes of the measles virus fusion protein have been described before (Steward et al. J. Virol. 69:7668, 1995).

Obeid et al. Virus Research 32 (1994), 69-84 have used peptides homologous with the measles virus hemagglutinin protein to investigate their ability to bind anti-MV serum and to induce MV-crossreactive antibodies. The overlapping 15mer peptides cover 92.22% of thehemagglutininsequence. Although these peptides were used for vaccinating BALB/c and TO mice, Obeid et al. concluded that although anti-MV antibodies raised in Balb/c and TO mice neutralized the virus in vitro (neutralization titer of 1:160), no neutralization was observed by the antipeptide antibodies. The peptides used would thus not have been considered in the preparation of a vaccine.

Linear binding sites of the MV-H specific antibodies have been mapped with sera from late convalescent donors by peptide ELISA using a full set of overlapping peptides of the MV-H protein (Muller et al. 1993).

It is therefore an object of the present invention to provide vaccines comprising peptides which are smaller than one of the naturally occurring structural proteins of a morbillivirus and which are capable of immunologically mimicking the morbillivirus.

It is a further object of the present invention to provide vaccines comprising peptides which are smaller than the naturally occurring proteins of the measles virus and which are capable of immunologically mimicking the measles virus hemagglutinin protein.

### Summary of the Invention

The present invention relates to the preparation of immunogens and candidate vaccines made of peptides containing amino acid sequences mimicking sequential antigenic determinants of the hemagglutinin protein of morbilliviruses in general and of the measles virus (MV) in particular.

Accordingly, in one aspect of the present invention, there is provided a vaccine comprising a peptide having an amino acid sequence corresponding to or mimicking at least one sequential antigenic determinant of the hemagglutinin protein of a morbillivirus, wherein the antigenic determinant is the binding site of a neutralizing and protective antibody of a morbillivirus.

This peptide in a vaccine alone or in combination with a T cell epitope is capable of eliciting a mammal to produce antibodies against said morbillivirus. Said peptide is either involved in the hemagglutination of Monkey Erythrocytes and/or in the neutralization of morbillivirus in vitro and/or in inducing a protection against a morbillivirus in vivo.

The antigenic determinant is, according to a preferred embodiment, a binding site of an in vitro neutralizing and in vivo protective antibody of a morbillivirus,

The protein may be the hemagglutinin (H) protein of a measles virus (MV). The peptide must preferably be recognized by an antibody which protects in vivo against the measles virus.

According to another preferred embodiment, said amino acid sequence comprises or mimicks at least a part of the amino acid sequence corresponding to amino acids 361 to 420 (HNE region) of the hemagglutinin protein of the Edmonston strain of MV or the corresponding domain of another morbillivirus, or a portion, variation, mutant or mimotope or conjugate thereof.

Preferably, said amino acid sequence comprises at least a part of the amino acid sequence corresponding to amino acids 381 to 400 of the hemagglutinin protein of the Edmonston strain of MV or the corresponding domain of another morbillivirus, or a portion, variation, mutant or mimotope or conjugate thereof.

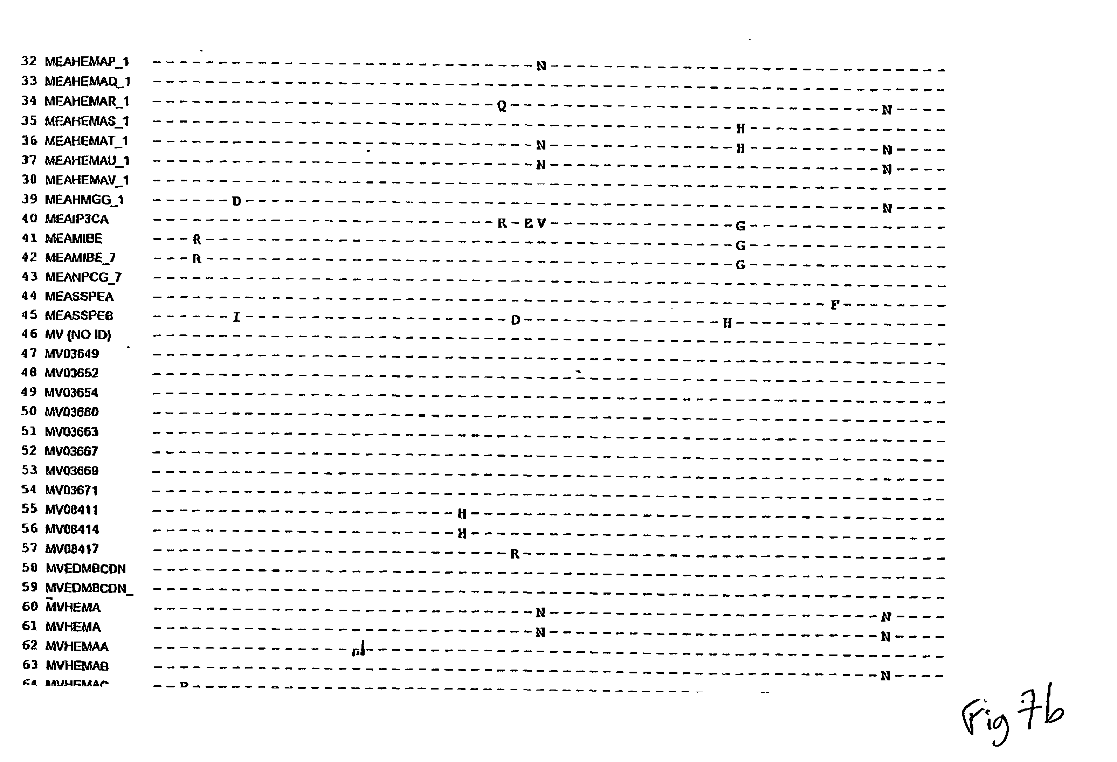

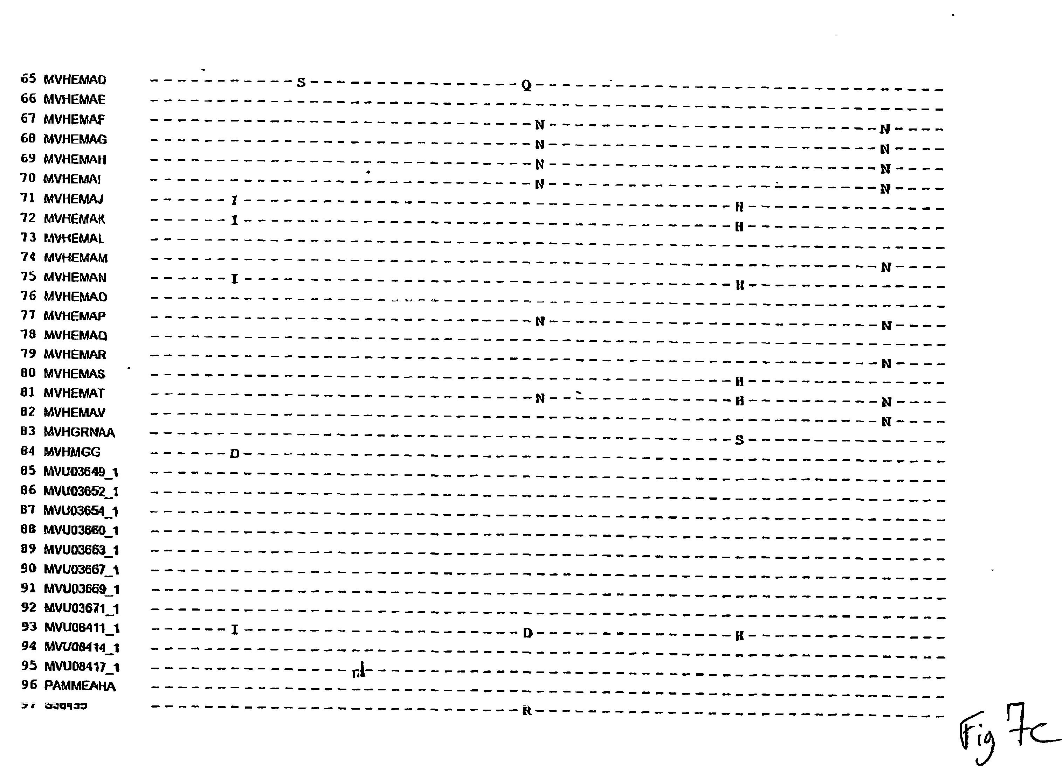

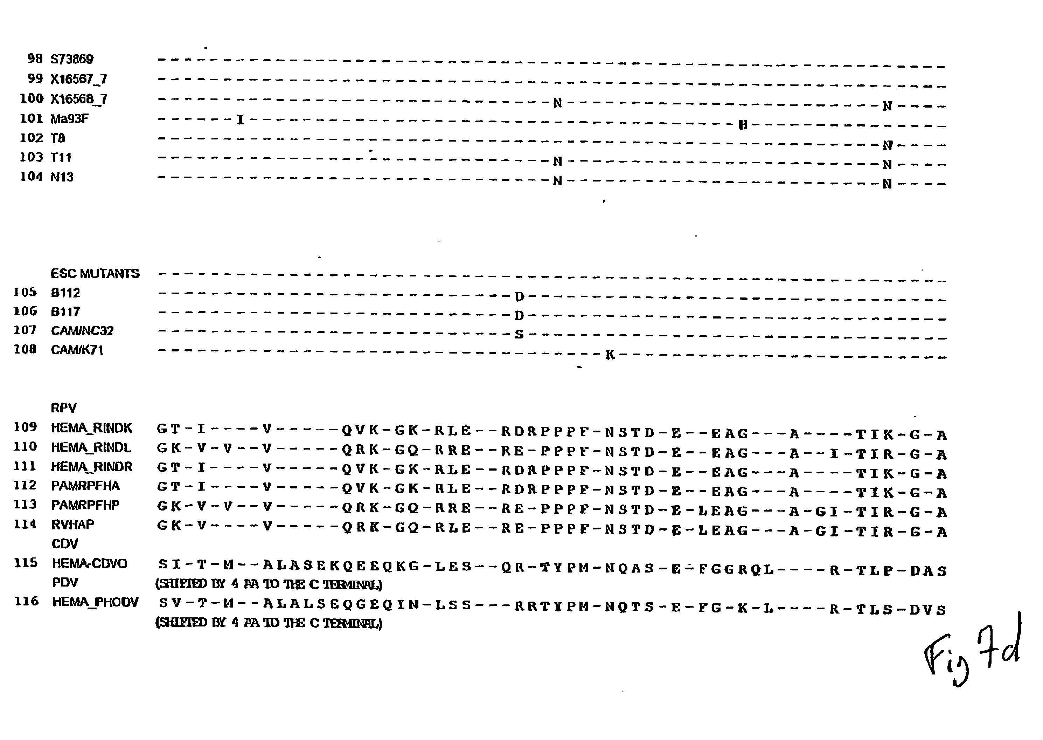

In another preferred embodiment, said amino acid sequence is selected from any of the amino acid sequences set forth in Fig. 7 or a portion, variation, mutant or mimotope or conjugate of any such sequence.

Said mutations may or may not involve one or more of the Cys residues.

Said peptide can be in an oxidized or a reduced form wherein said oxidized form has disulfide bridges between sulfur-containing amino acids. The oxidized form is probably more active, than the reduced form but the reduced form is also active.

According to a further preferred aspect of the present invention, there is provided a vaccine comprising a peptide wherein said amino acid sequence comprises at least a part of the amino acid sequence corresponding to amino acids 226 to 255 of the hemagglutinin protein of the Edmonston strain of MV or the corresponding domain of another morbillivirus, or a portion, variation, mutant or mimotope or conjugate thereof.

Said peptide comprises preferably an amino acid sequence that corresponds to. amino acids 241 to 251 of the hemagglutinin protein of the Edmonston strain of MV or the corresponding domain of another morbillivirus, or a portion, variation, mutant or mimotope or conjugate.

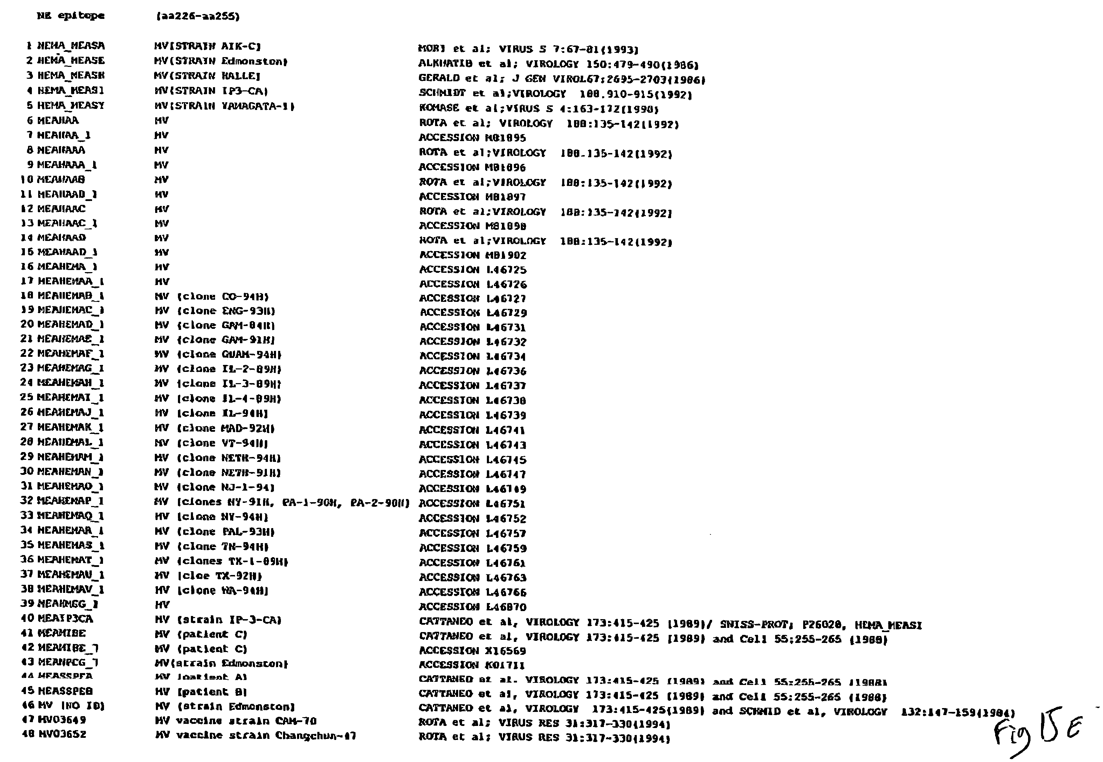

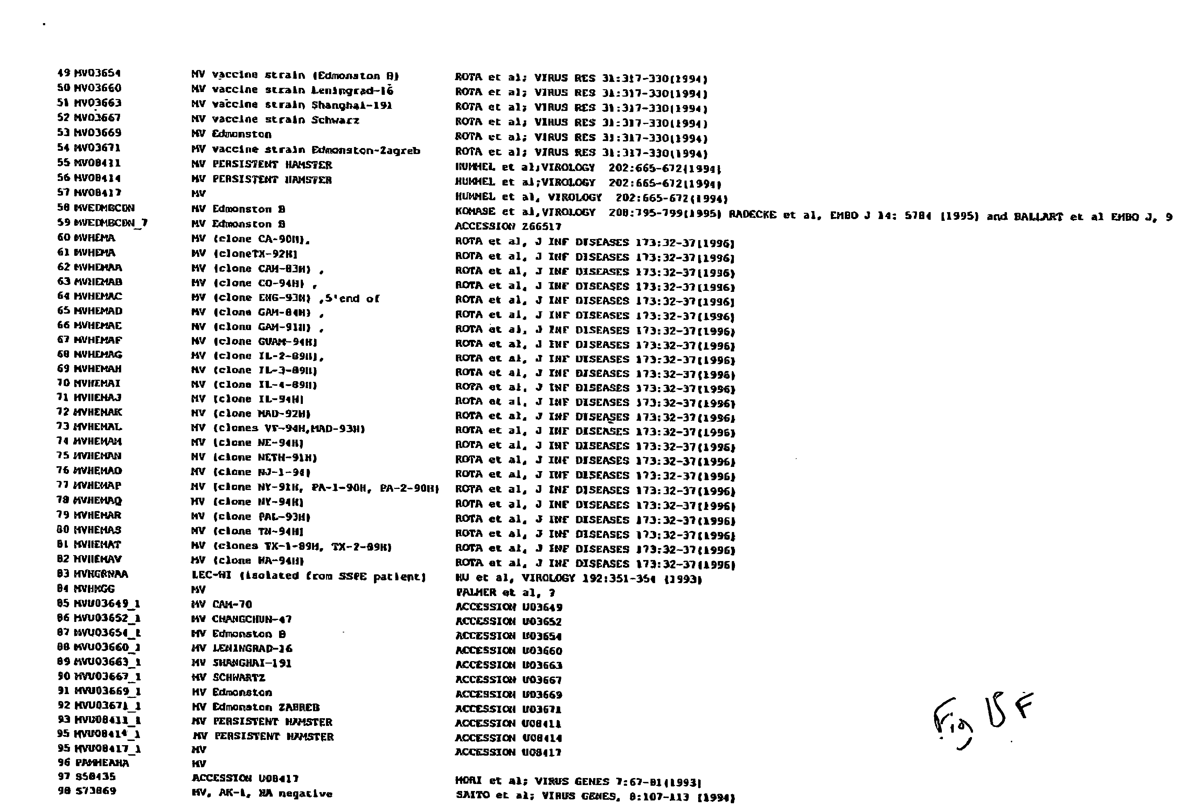

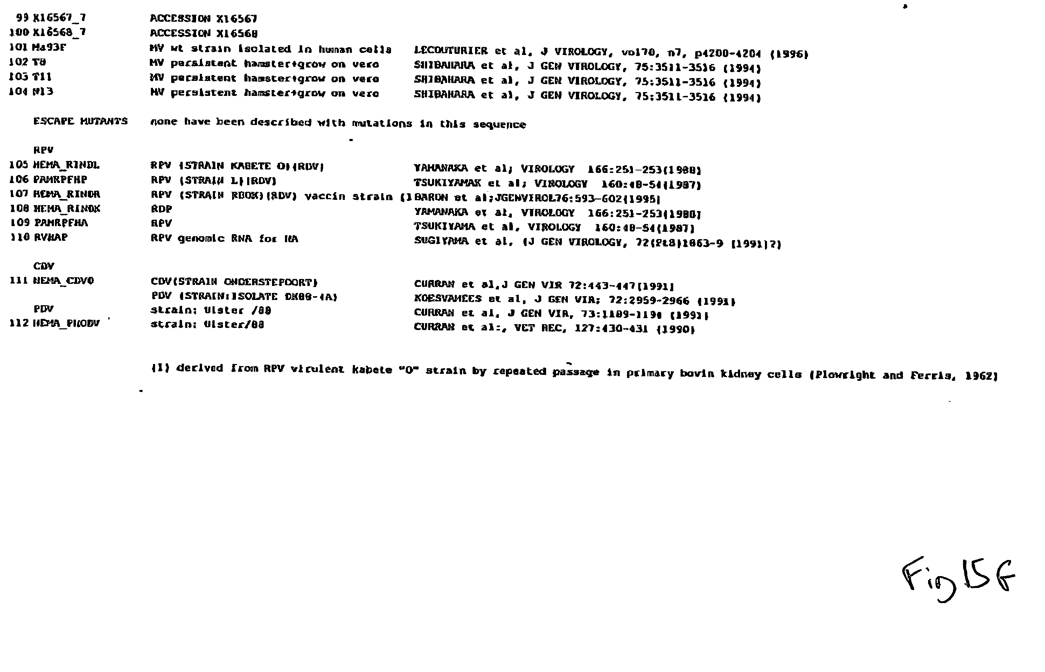

In yet another preferred embodiment, said amino acid sequence is at least one selected from any of the amino acid sequences set forth in Fig. 15-18 or a portion, variation, mutant or mimotope or conjugate of any such sequence.

The peptide has preferably less than 60 amino acid residues and most preferably less than 30 amino acid residues.

In one aspect of the invention, the vaccine is suitable for the prevention of measles.

The vaccine may comprises the peptide in an amount of about 0.1 micrograms to about 100 milligrams and further comprises, if necessary, a pharmaceutically acceptable diluent, adjuvant, carrier, T cell epitope or excipient.

In another aspect of the invention the peptide as described above is used in the manufacture of a medicament for the prevention and/or treatment of measles.

In a further aspect of the invention, there is provided a sequential antigenic determinant comprising at least a part of the amino acid sequence corresponding to amino acids 226 to 255 and/ or 361 to 410 of the hemagglutinin protein of the Edmonston strain of MV or a portion, variation, mutant or mimotope or conjugate of any such sequences. The invention also comprises peptides comprising at least one such sequential antigenic determinant.

The peptide may also be used as a medicament in the prevention and/or treatment of a disease caused by a morbillivirus in general or for the prevention and/or treatment of measles in particular.

### Description of the Drawings

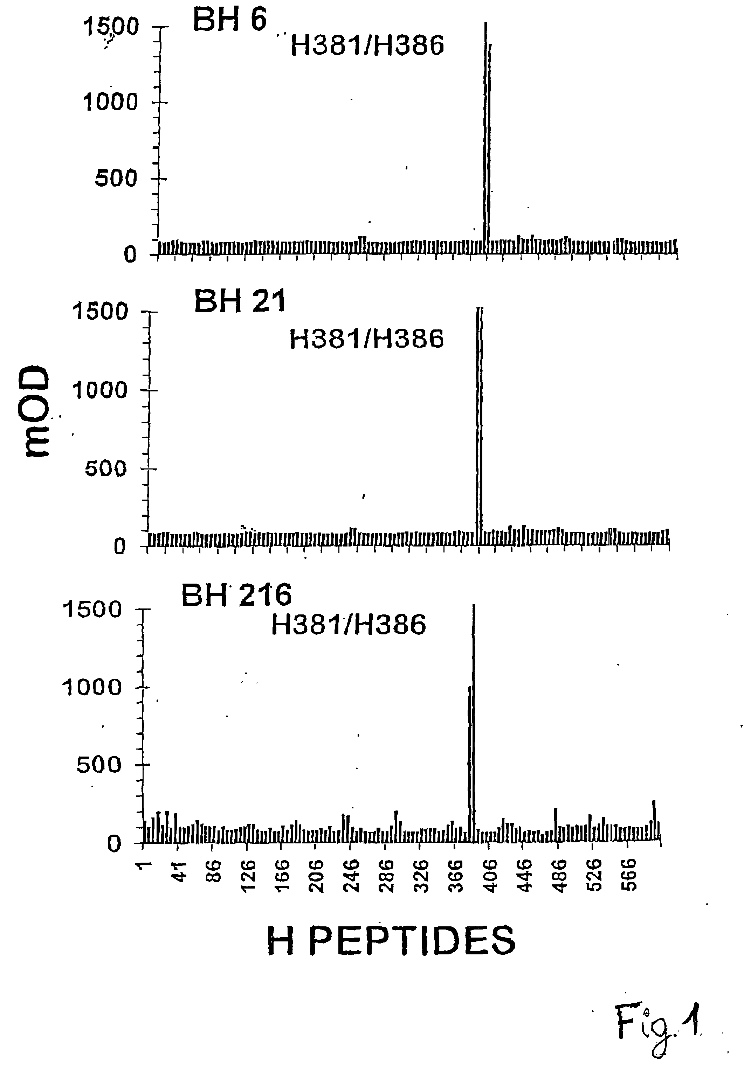

**Fig. 1.** shows the binding of selected mAbs (supernatant dilution 1:20) with 121 overlapping biotinylated 15mer peptides covering the whole sequence of the MV-H protein. The peptides were immobilized by their C-terminal biotin on streptavidin coated microtiter plates. The absorbance is expressed in mO.D.₄₀₅ Numbers along the x-axis indicate the position in the MV-H sequence of the N-terminal amino acid of the peptides.

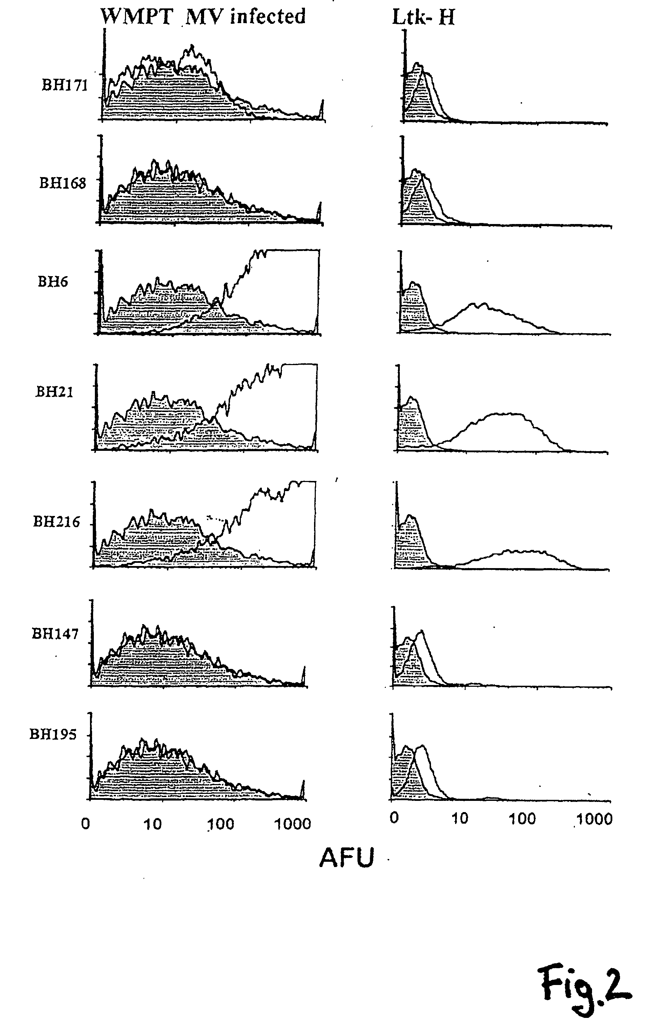

**Fig. 2.** shows the binding of mAbs to MV-infected EBV-transformed human B cells (WMPTT; left panels) and Ltk-H cells (right panels) measured by flow cytometry. Background binding corresponds to the FITC-conjugate alone on EBV-MV cells (GaM) or Ltk-H cells. The data are expressed on a log-scale (3 decades) as arbitrary fluorescence units (AFU). Background binding of mAbs to the uninfected EBV cells and to Ltk⁻ wild-type cells was similar to the background of GaM on the corresponding H expressing cells (data not shown).

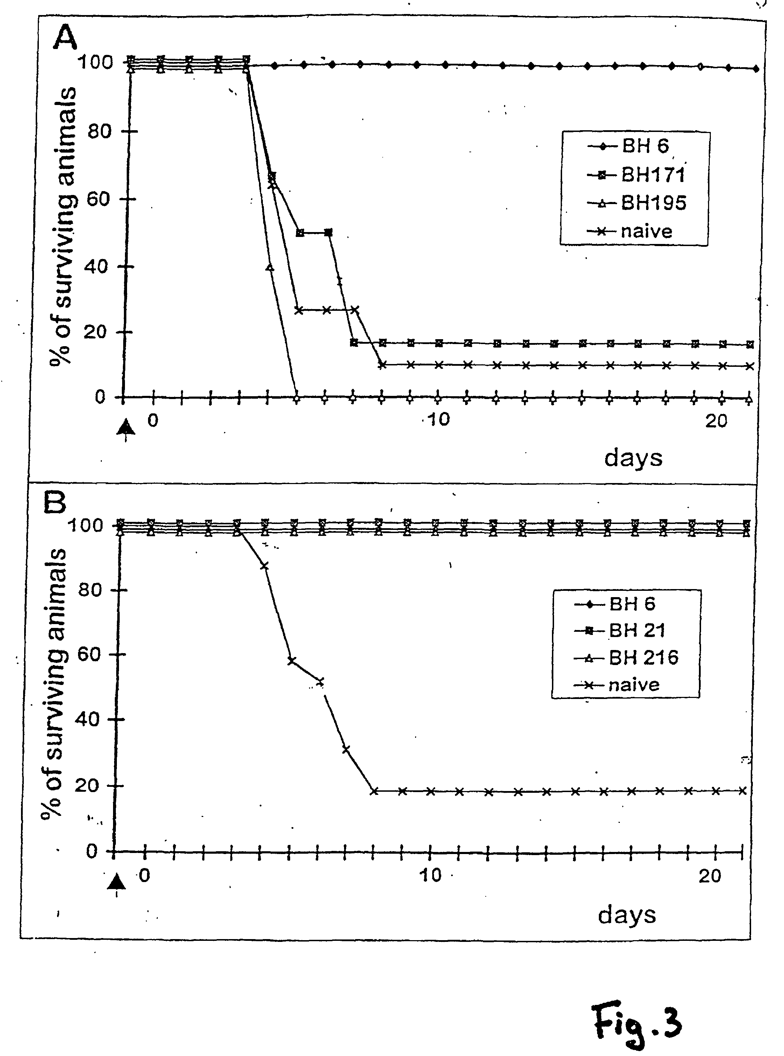

**Fig. 3.** shows a challenge/protection experiment with mice passively immunized on day -1 with 315 µg BH6, 344 µg BH171, 330 µg BH195 (panel A) or 62±6 µg BH6, BH21 or BH216 (panel B). On day 0, mice were challenged with an intracranial injection of CAM mouse adapted MV. Naive mice were challenged without prior antibody transfer. Each group represents 6 to 8 mice from 2 independent experiments.

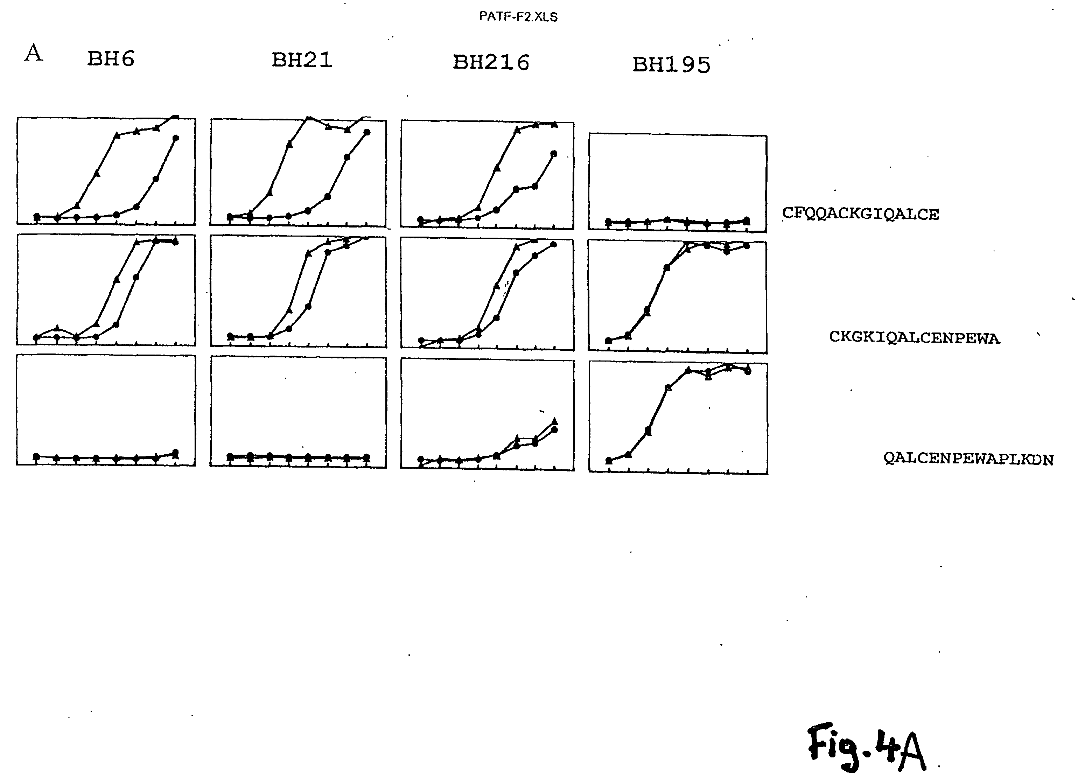

**Fig. 4.** represents the titration of mAb BH6, BH21, BH195 and BH216 on biotinylated peptide analogues of H386 immobilized to streptavidin coated plates. Highest concentrations of mAbs used: BH6, 10 µg/ml; BH21 3.5 µg/ml; BH216 11.2 µg/ml; BH195 2.6 µg/ml. Fig 4A: peptide H386 (15mer) and adjacent 15mers H381 and H391. Fig 4B: amino-butyric acid (X) single and double substitution analogues of H386: H386(Cys_{386/394}-->Abu), H386(Cys₃₈₆-->Abu), H386(Cys₃₉₄-->Abu). Fig 4C: C-terminal truncation and elongation analogues of H386. ▲, oxidized peptides; ●, reduced peptides. The data is shown as O.D.405 on the Y-axis. the scale ranging for all panels from 0 to 2.0. The X-axis shows the dilution ranging in four-fold dilution steps from undiluted to 1:16384 of the above stock solutions.

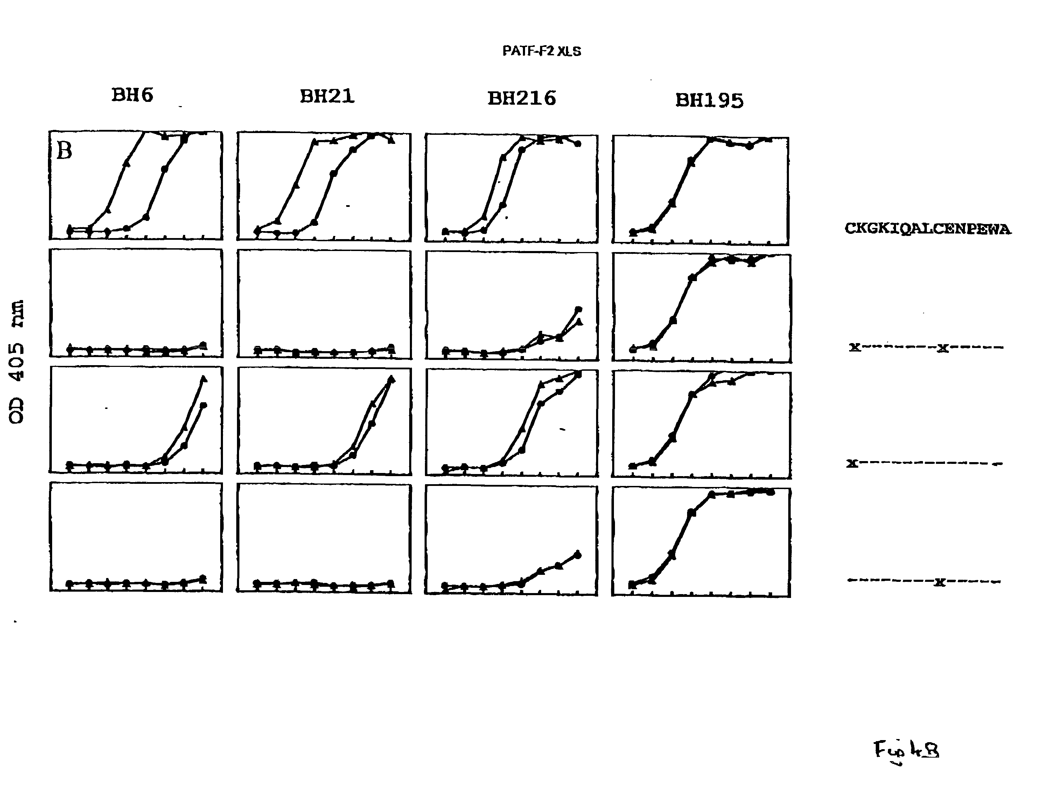

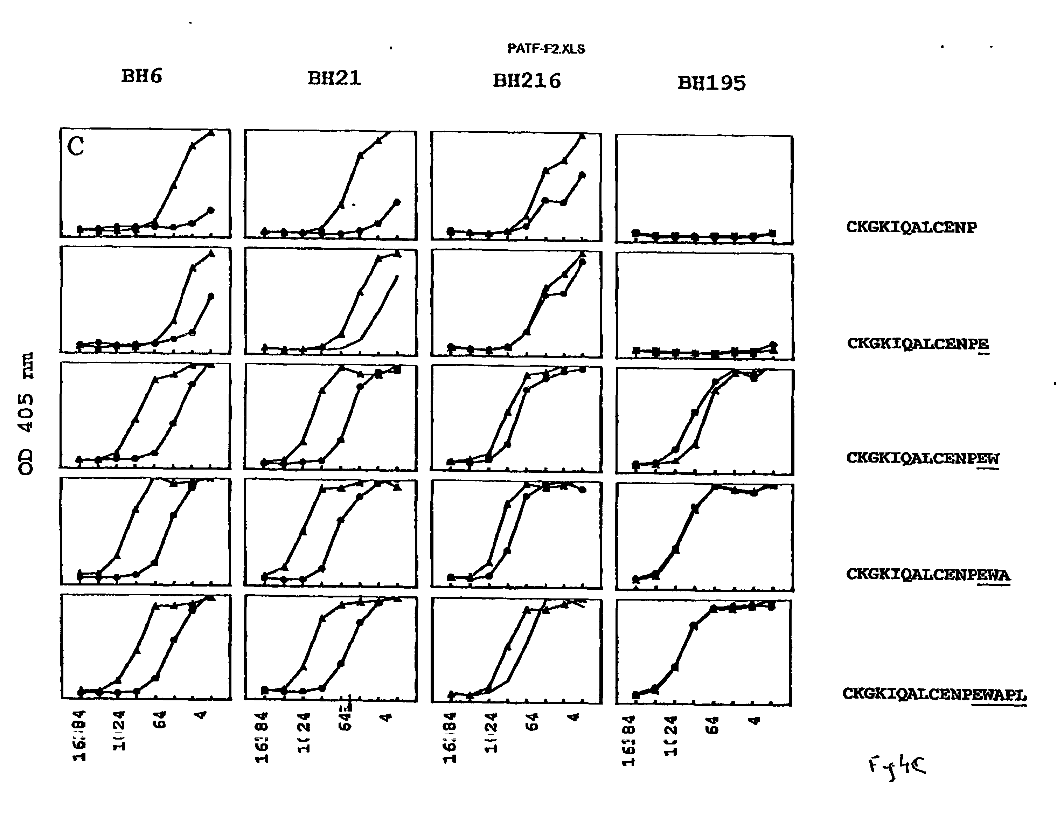

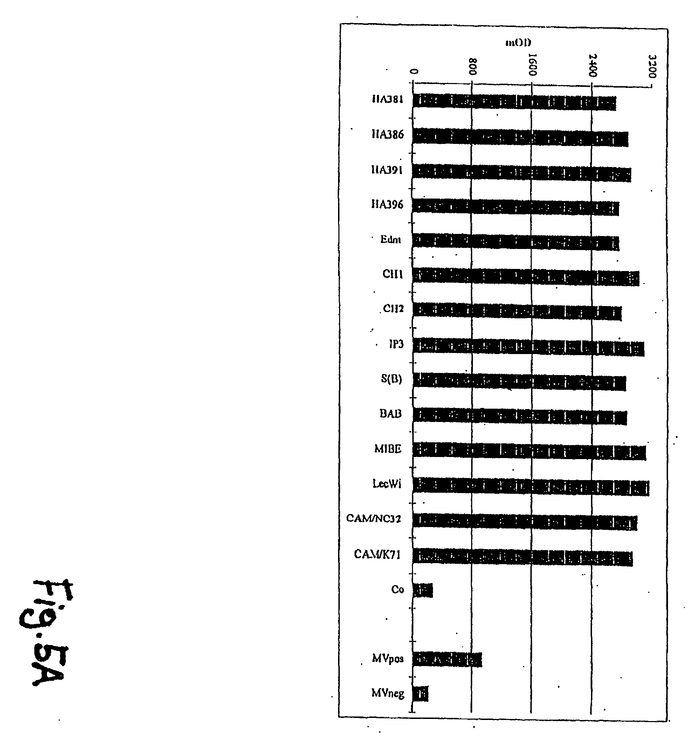

**Fig. 5.** A shows the reactivity of mouse sera (dilution 1:100) with selected 15mer (H381, H386, H391, H396) and 30mer peptides (EDM, CH1, CH2 IP3, S(B), BAB, MIBE, LecWi, CAM/NC32, CAM/K71) and MV (Mvpos) after immunization with the H381 (30mer) peptide of the Edmonston strain.
**Fig. 5.** B shows the binding of serum of a mouse immunized with 25 ug H386 (15mer) coupled to KLH-Maleimid CFA/IFA to MV-infected EBV-transformed human B cells (WMPTT) measured by flow cytometry. Background binding corresponds to serum reactivity on non-infected cells. The data are expressed on a log-scale (3 decades) as arbitrary fluorescence units (AFU).

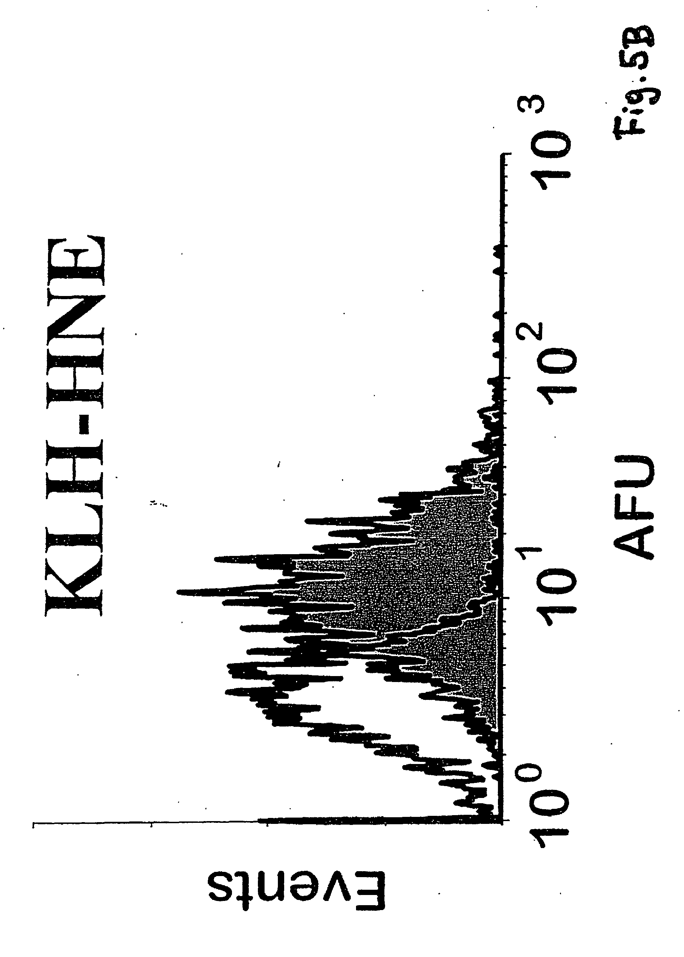

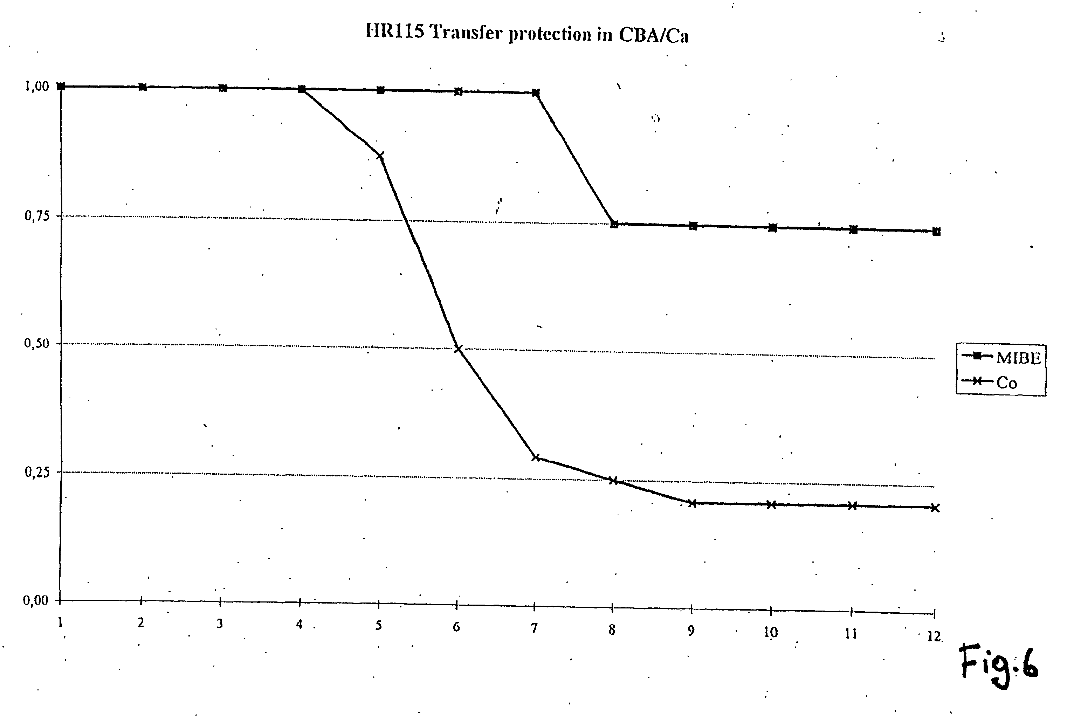

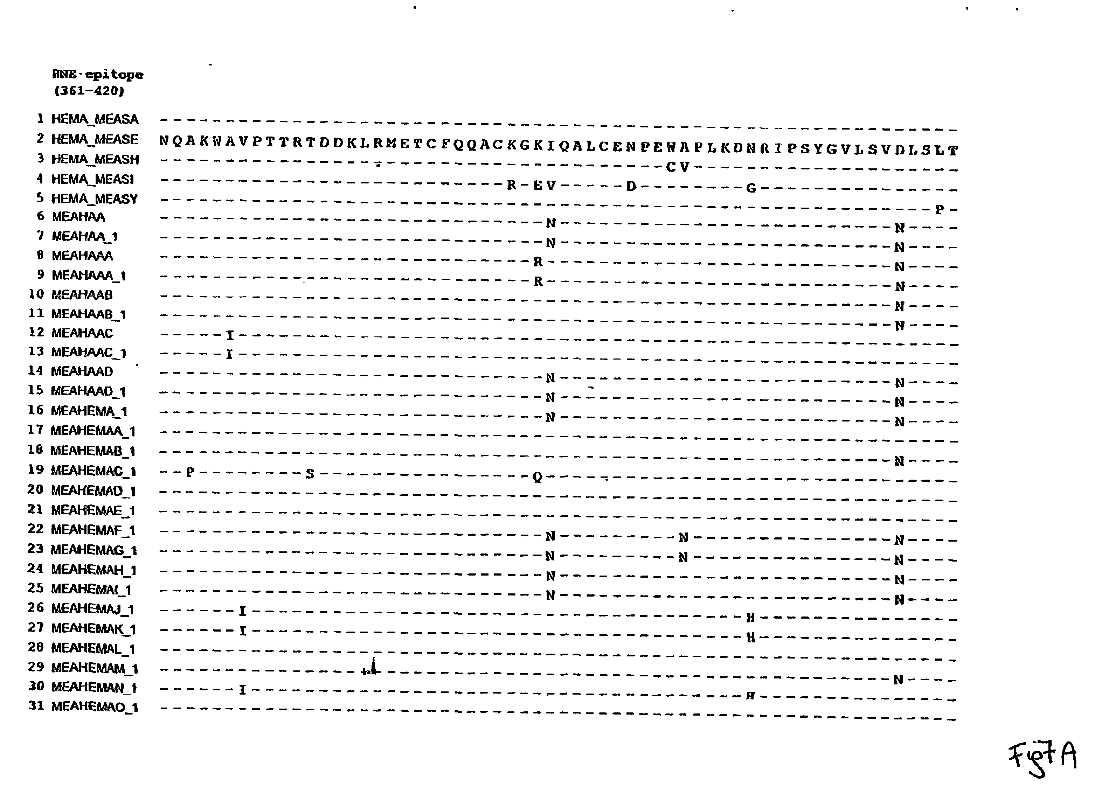

**Fig.6.** shows the survival of CBA mice after passive transfer of serum (100 µl; day - 1) from mice immunized with a 30mer peptide and challenge with a lethal dose of rodent adapted measles virus (day 0). Control mice were mock immunized with CFA/IFA only and challenged in the same way.
**Fig 7.** shows the alignment of the hemagglutinin neutralizing epitope (HNE) region (amino acid 361-420 of MV-H) of different MV isolates and of in vitro-induced antibody escape mutants and of other morbillivirus. Only amino acids differing from the Edmonston wild-type strain (EDWT, Alkhatib & Briedis, 1986) are shown.

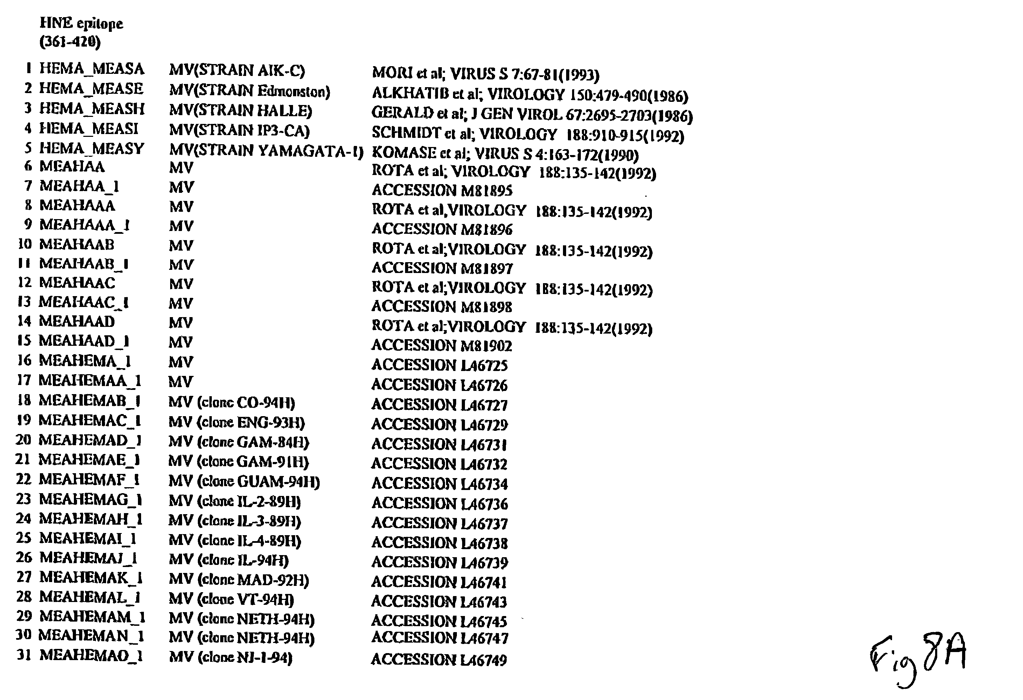

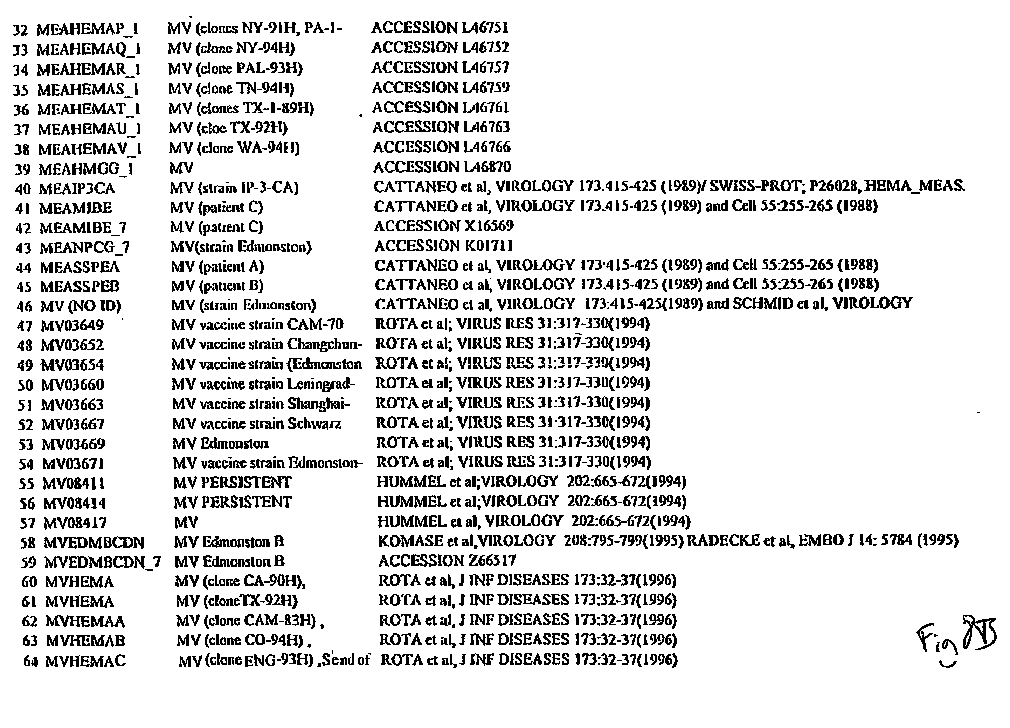

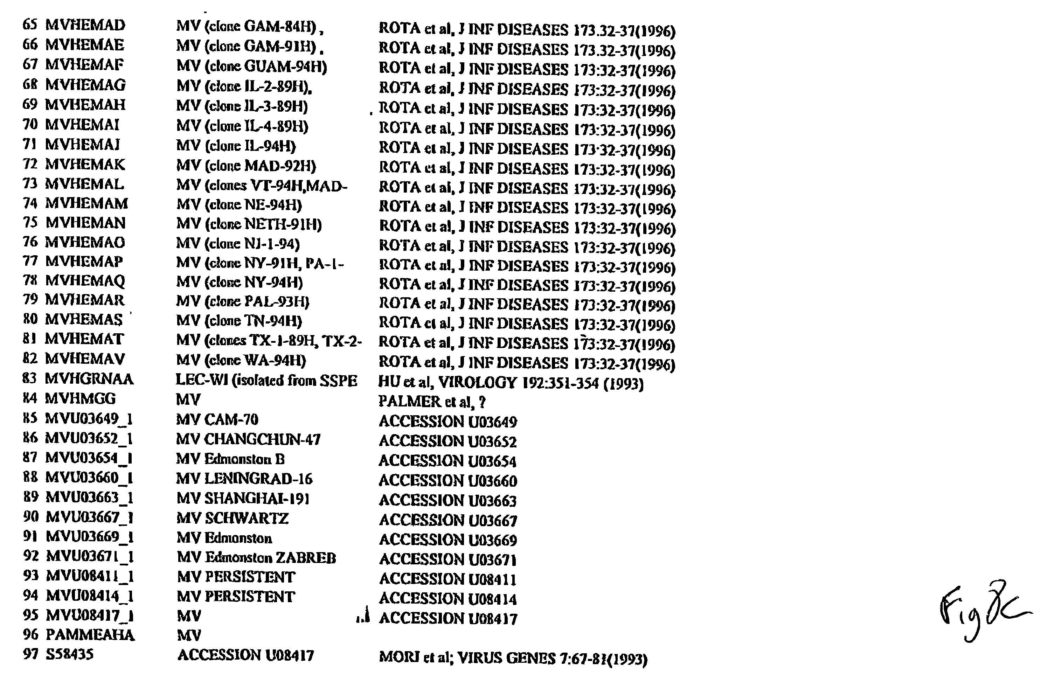

Fig 8. shows the description of the measles virus sequences shown in Figure 7.

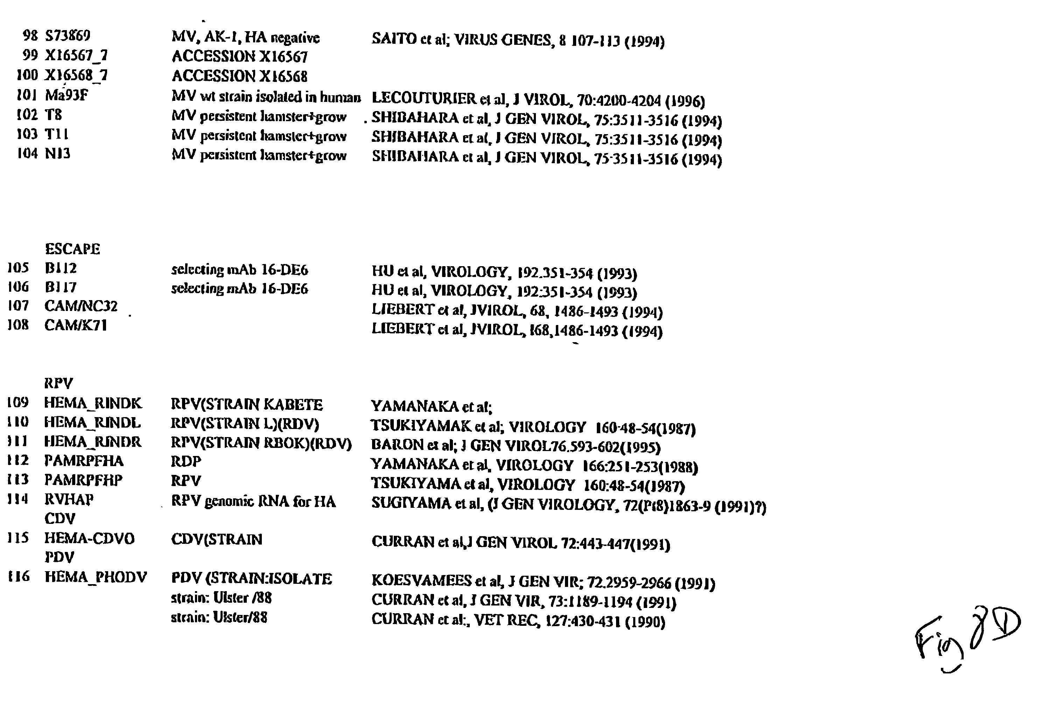

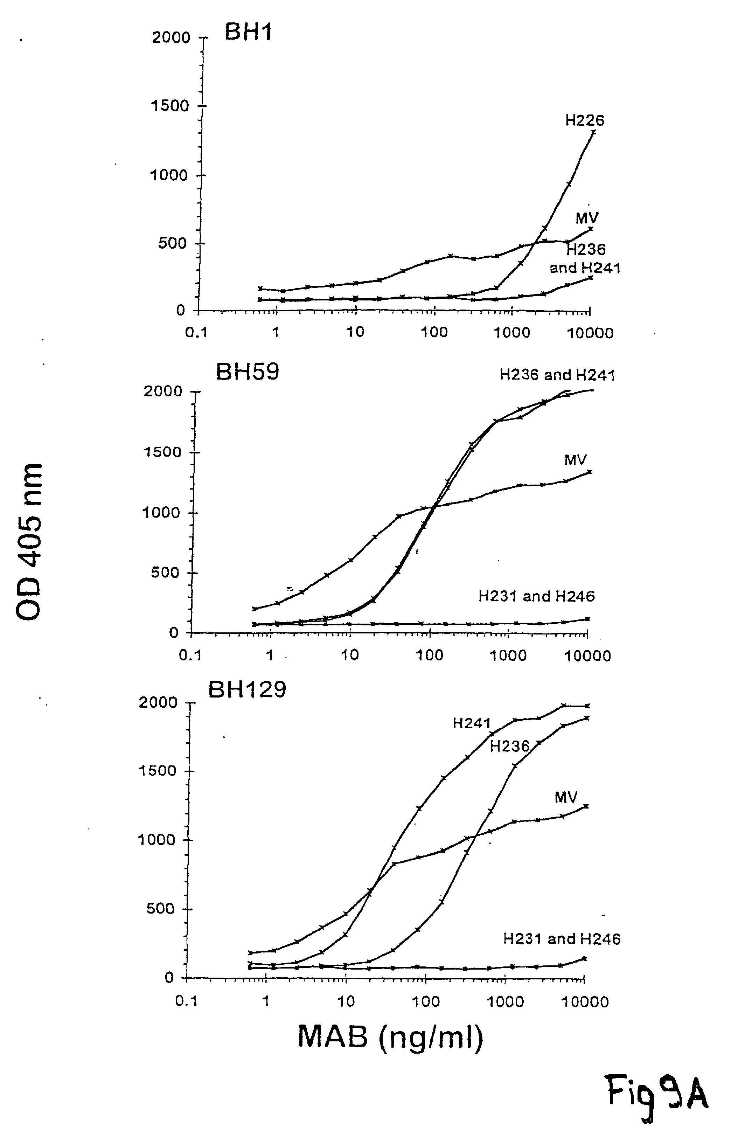

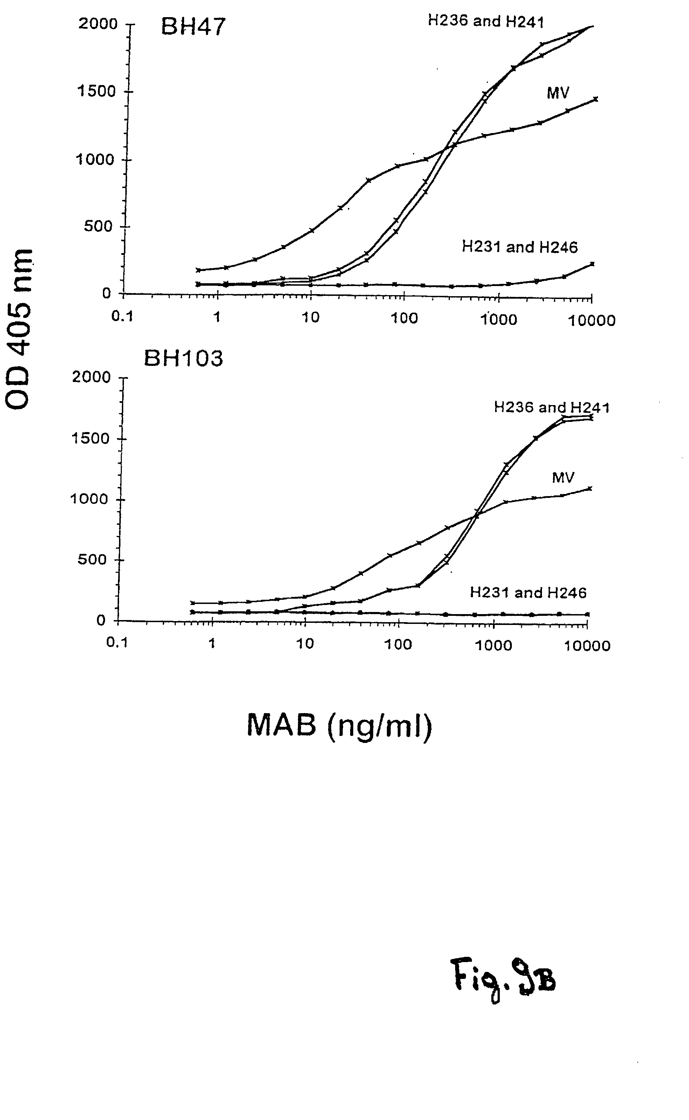

**Fig. 9A and Fig. 9B.** Titration of mAbs against peptides (mAb BH1: H226, H236, H241; other mAbs: H231, H236, H241, H246) and MV by ELISA.

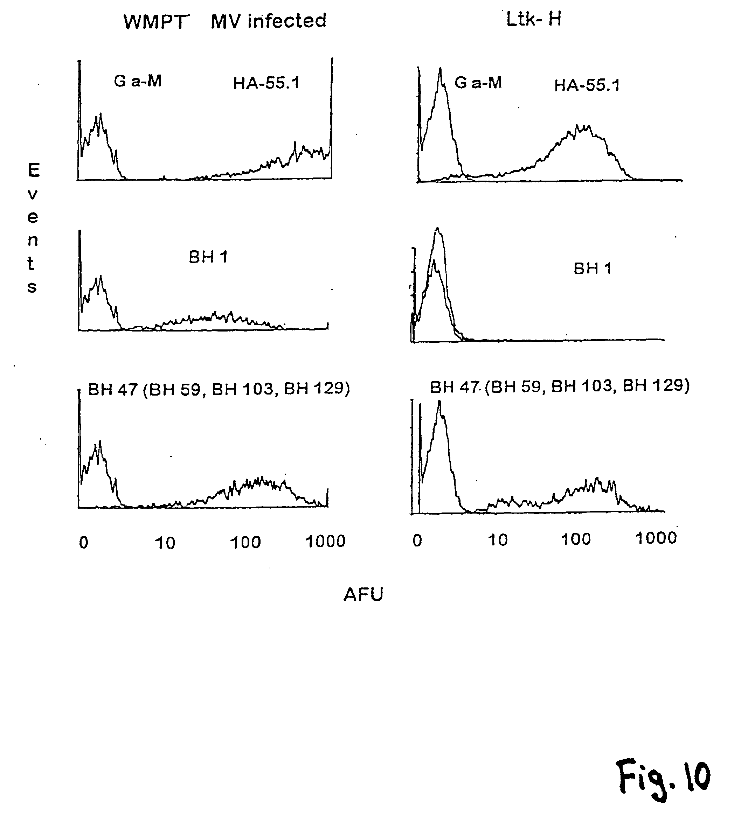

**Fig. 10.** shows the binding of mAbs to MV-infected EBV-transformed human B cells (WMPT; left panels) and Ltk-H cells (right panels) measured by flow cytometry. Background binding corresponds to the FITC-conjugate alone on EBV-MV cells (GaM) or Ltk-H cells. The data are expressed on a log-scale (3 decades) as arbitrary fluorescence units (AFU). Background binding of mAbs to the uninfected EBV cells and to Ltk⁻ wild-type cells was similar to the background of GaM on the corresponding H expressing cells (data not shown).

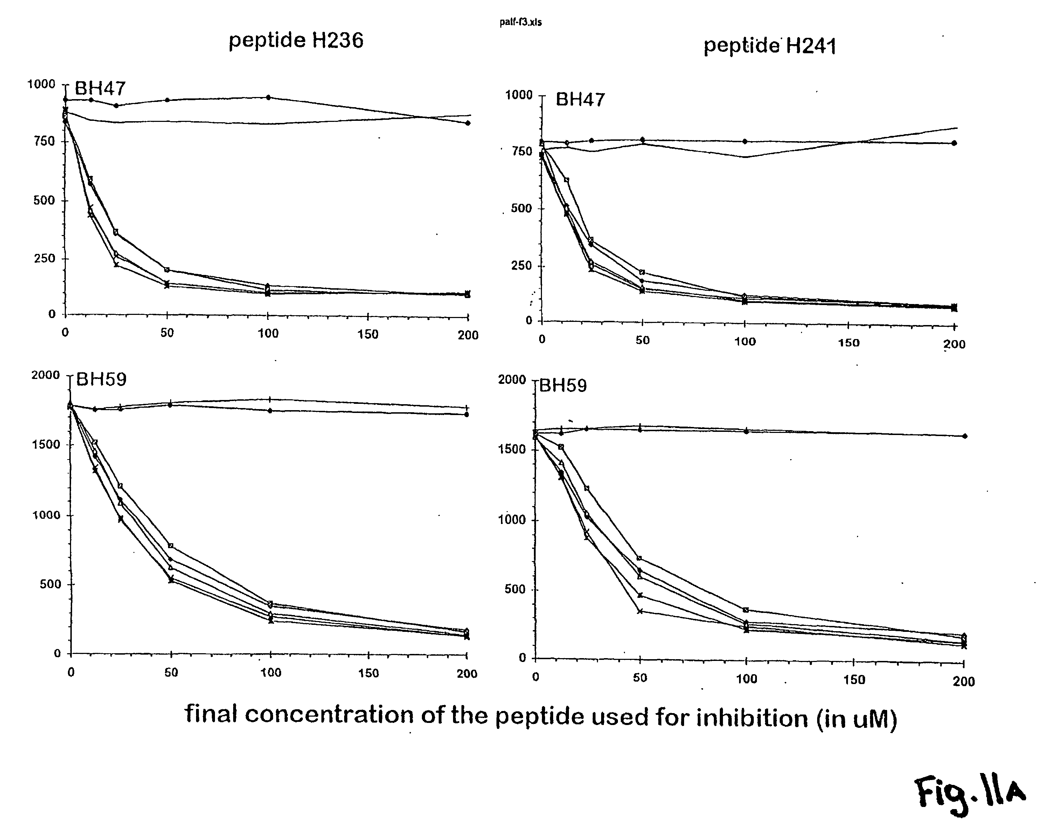

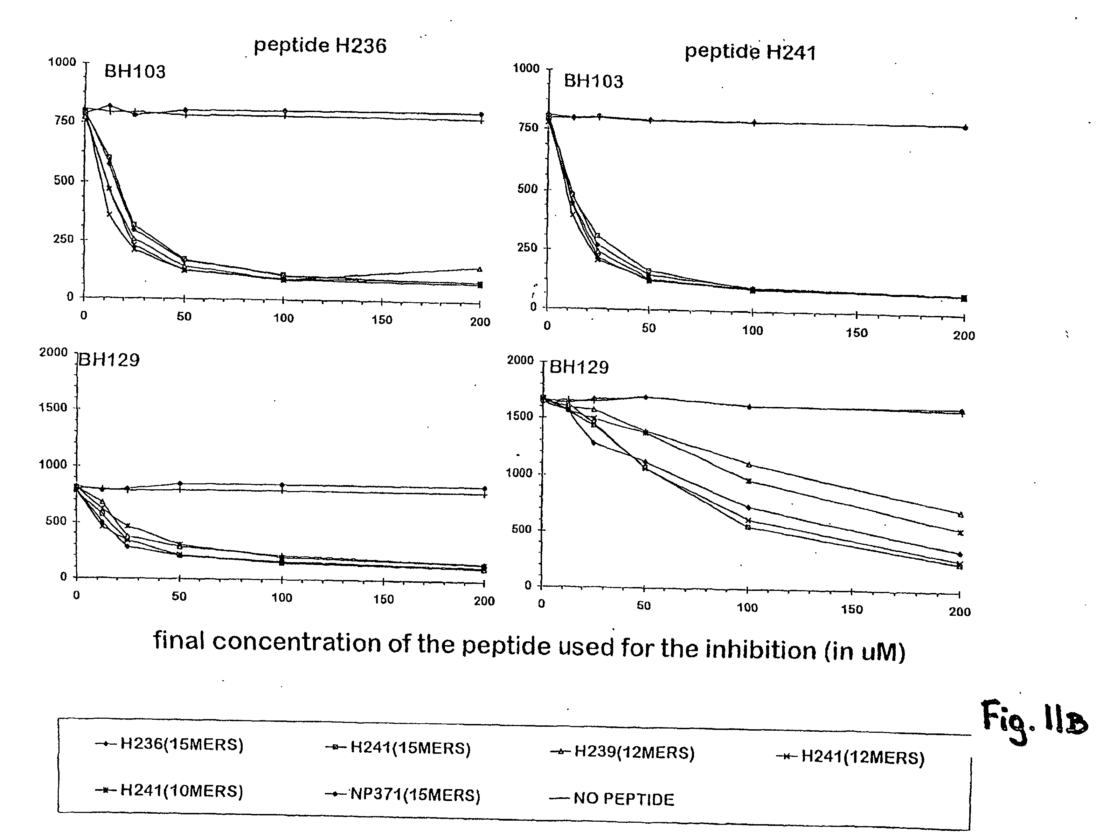

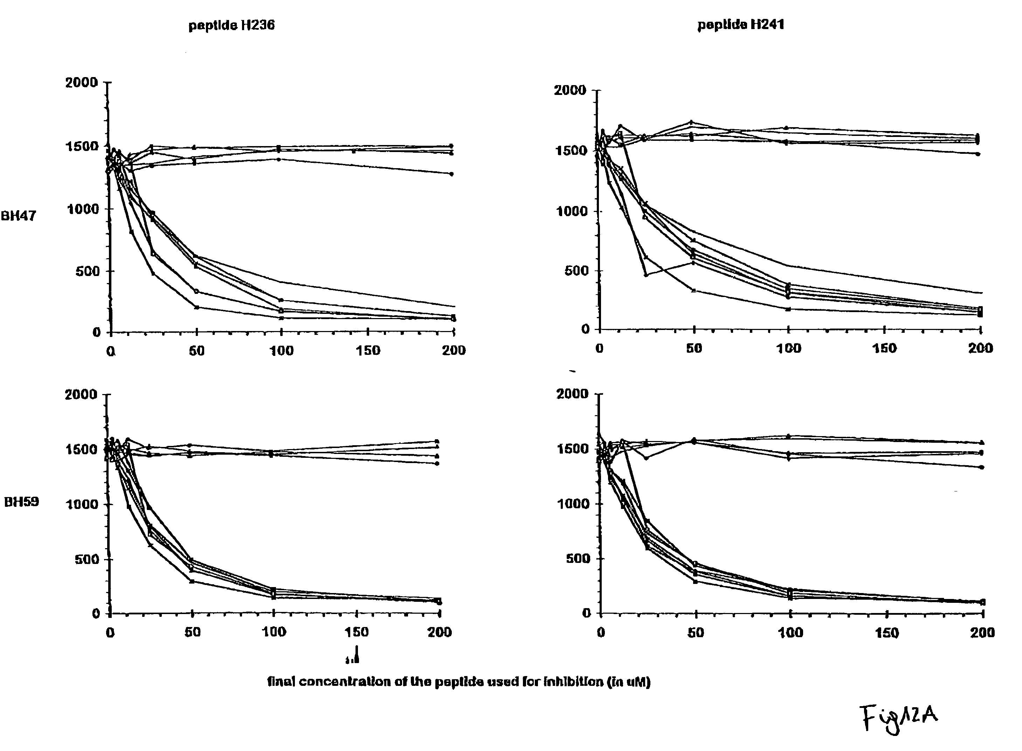

**Fig. 11A and Fig. 11B.** Inhibition of mAb binding to immobilized H236 (left panels) and H241 (right panels) by increasing concentrations of truncation analogues (15mers, 12mers, and 10mer). The irrelevant peptide NP371 or water served as negative controls.

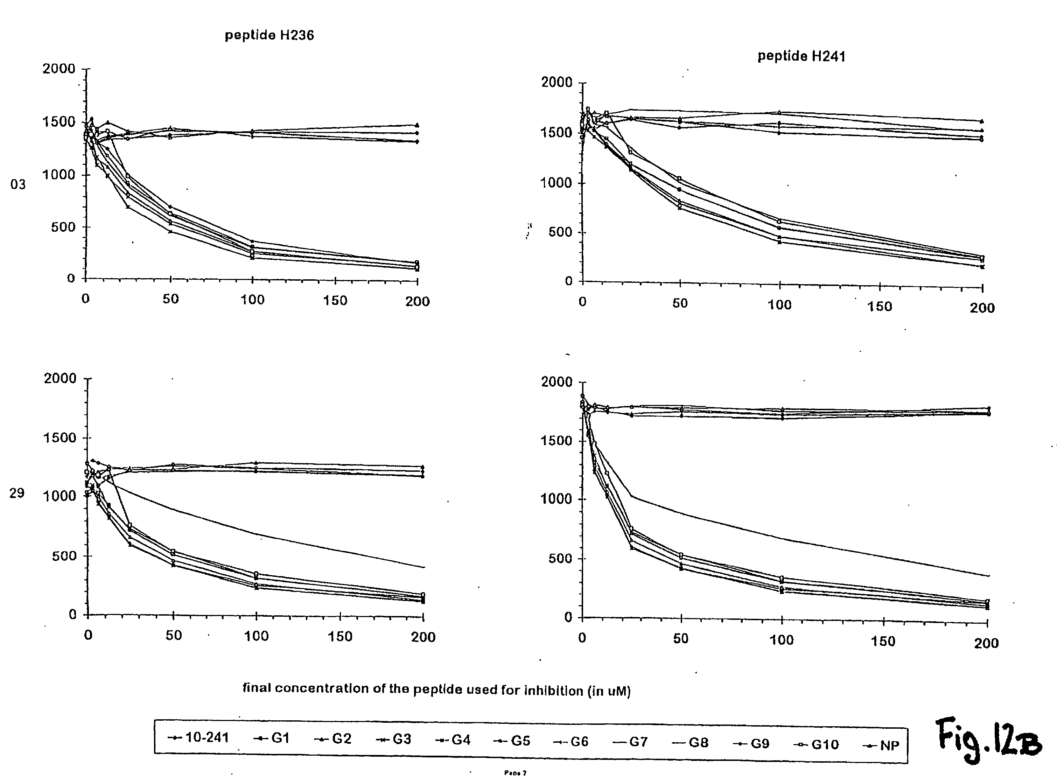

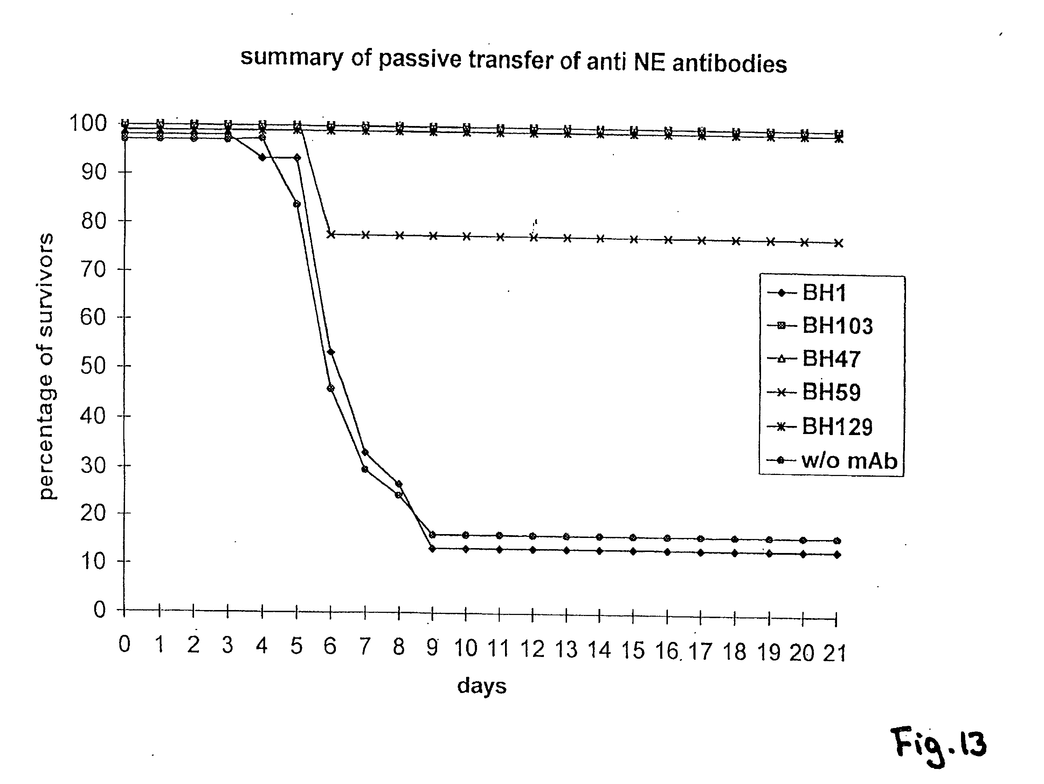

**Fig. 12A and Fig. 12B.** Inhibition of mAb binding to immobilized H236 (left panels) and H241 (right panels) by increasing concentrations of glycin monosubstitution analogues. H241 (10mer) (10-241) served as positive competition control. G1 corresponds to H241 substituted in position 1 by glycin, etc. The irrelevant peptide NP371 served as negative controls.

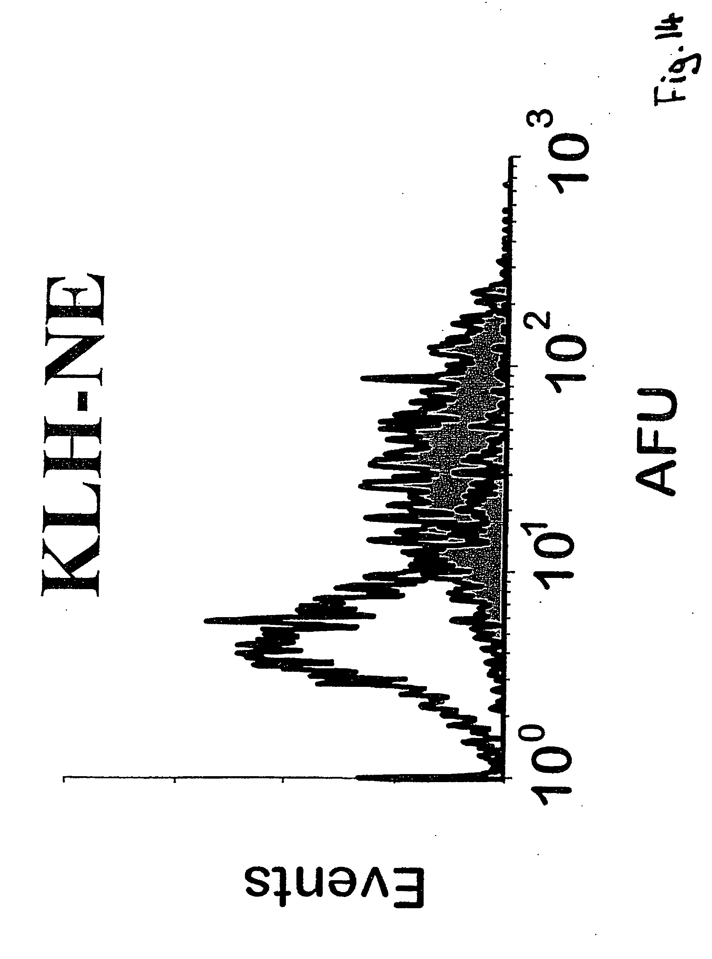

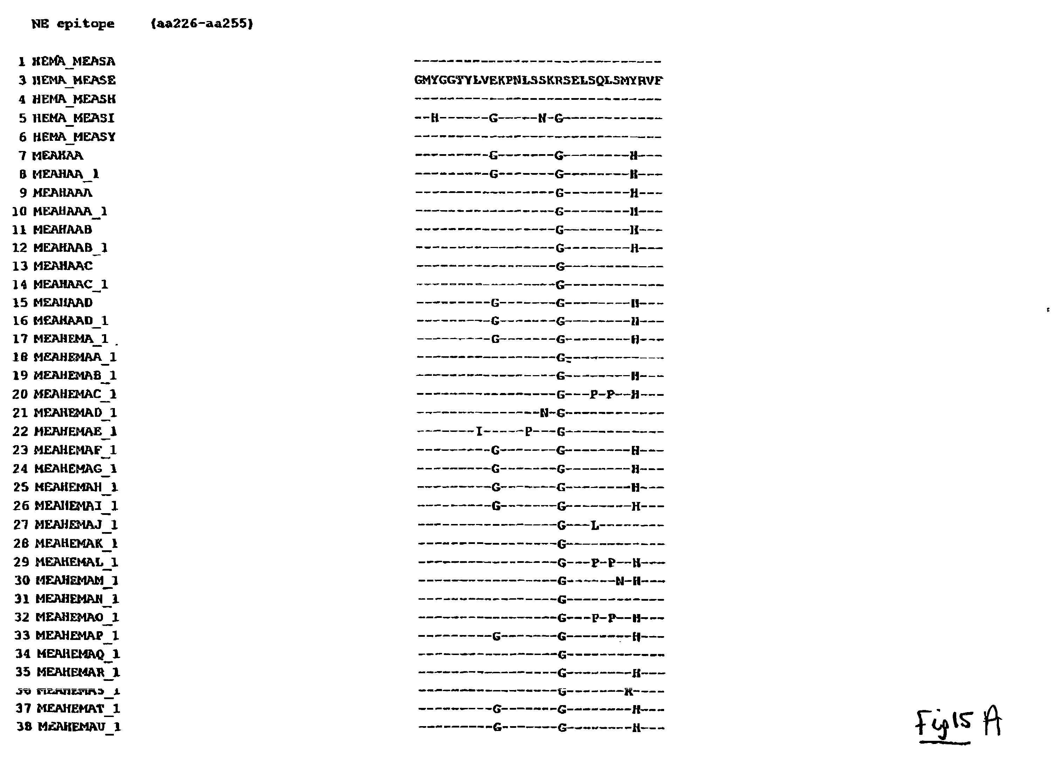

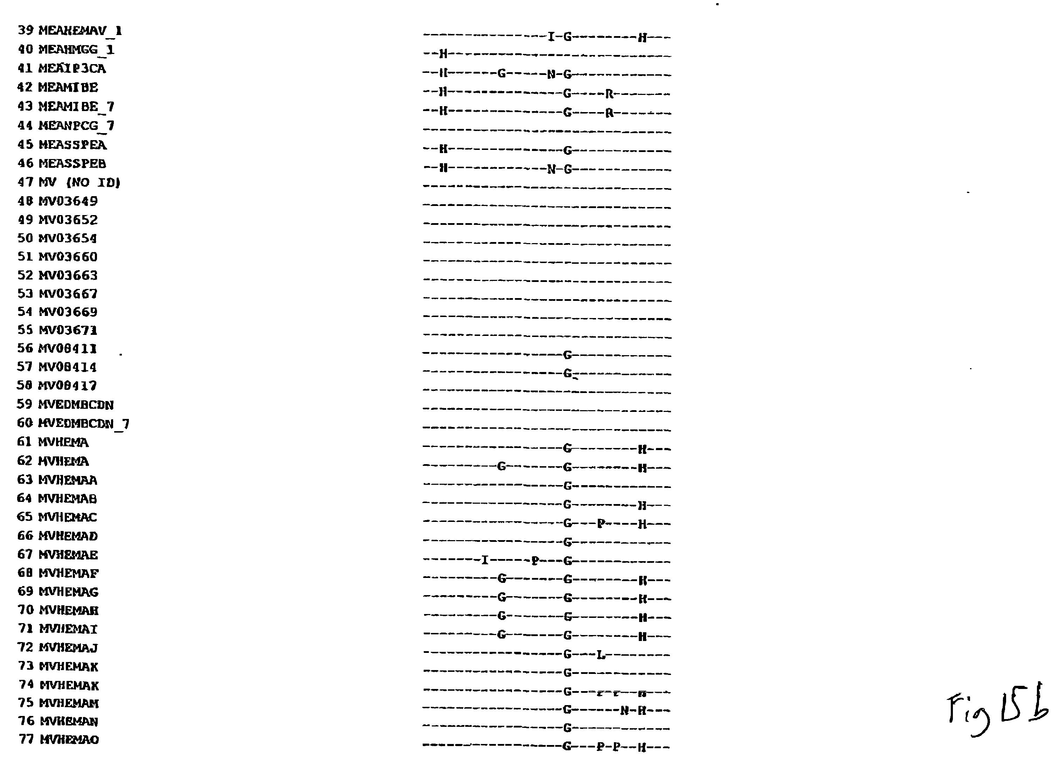

**Fig.13.** shows a challenge/protection experiment with mice passively immunized on day -1 with BH1 (275-700 µg), BH47 (92-368 µg), BH59 (366 µg), BH103 (74 µg), BH129 (368 µg). On day 0, mice were challenged with an intracranial injection of CAM mouse adapted MV. Naive mice were challenged without prior antibody transfer. Each group represents 6 to 8 mice from 2-3 independent experiments.

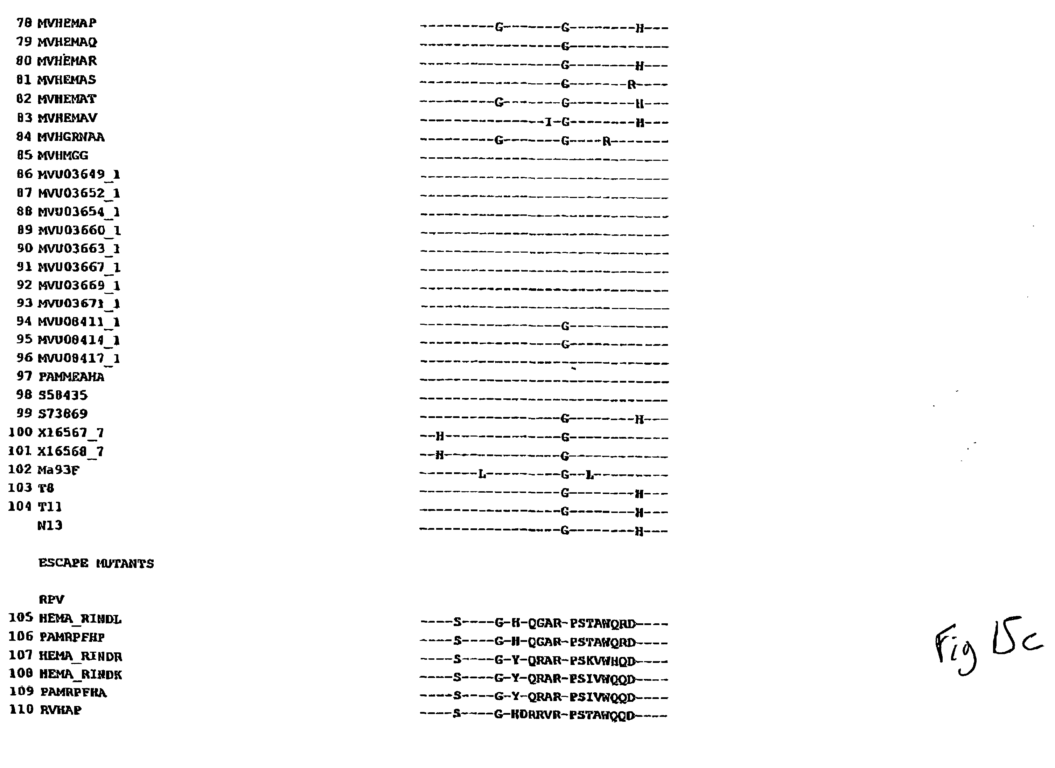

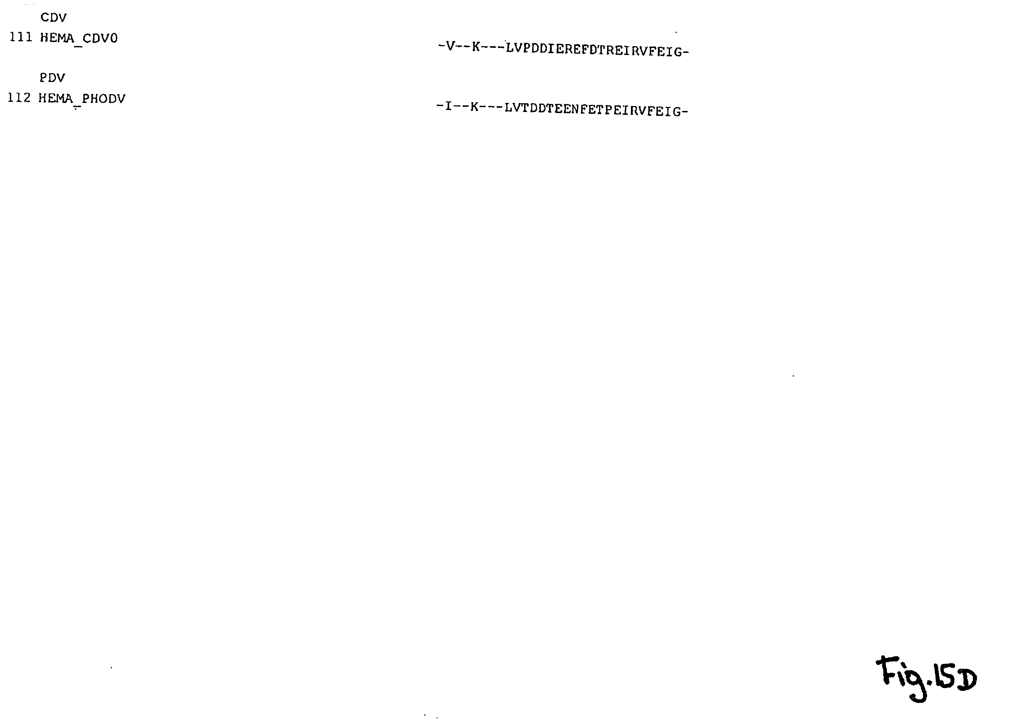

**Fig.14.** shows the binding of serum of a mouse immunized with 25 ug H236 (15mer) coupled to KLH-Maleimid CFA/IFA to MV-infected EBV-transformed human B cells (WMPT) measured by flow cytometry. Background binding corresponds to serum reactivity on non-infected cells. The data are expressed on a log-scale (3 decades) as arbitrary fluorescence units (AFU).
**Fig. 15A-G.** shows the alignment of the hemagglutinin neutralizing epitope (NE) region (amino acid 226-255 of MV-H) of different MV isolates and of in vitro-induced antibody escape mutants and of other morbilliviruses. Only amino acids differing from the Edmonston wild-type strain (EDWT, Alkhatib & Briedis, 1986) are shown. EG shows the description of the measles virus sequences shown in A-D.

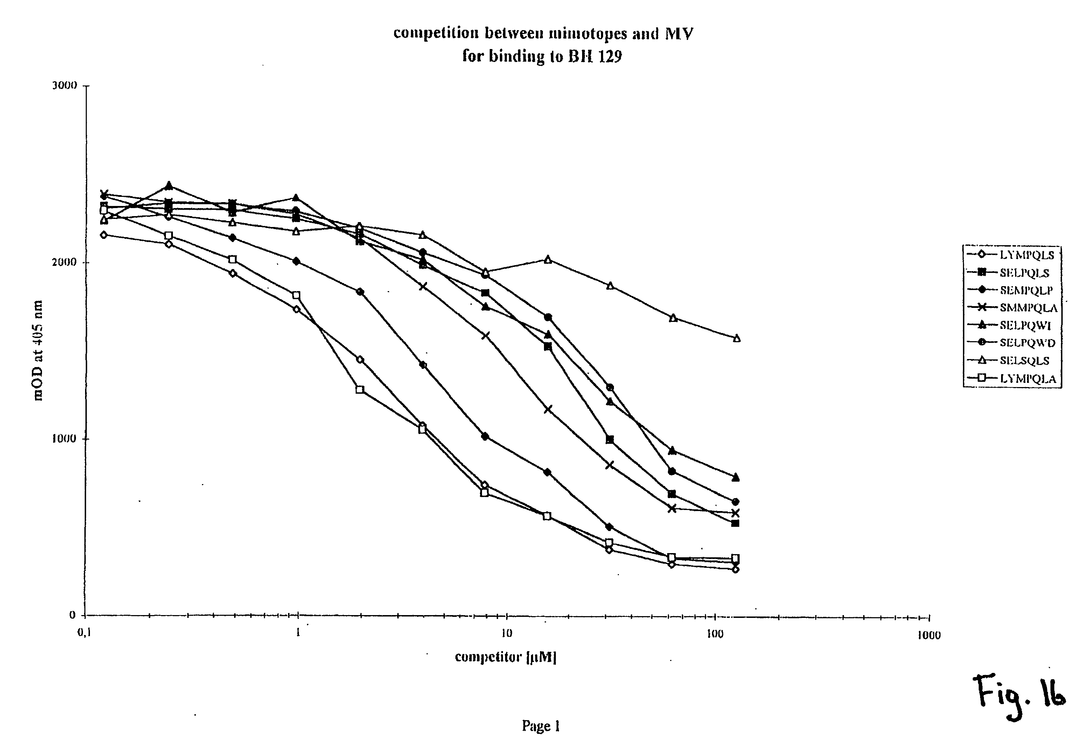

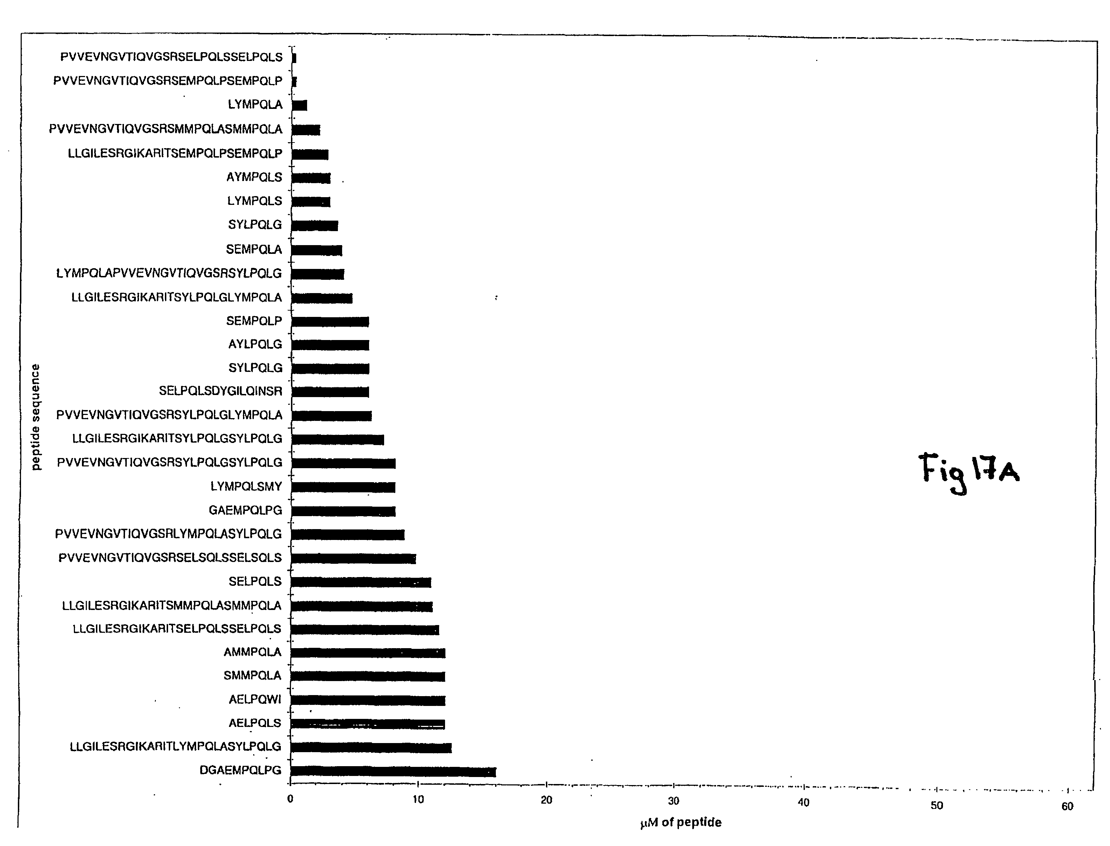

**Fig. 16** shows the inhibition of binding of saturating concentrations of BH129 to immobilized measles virus by mimotopes (competitor).

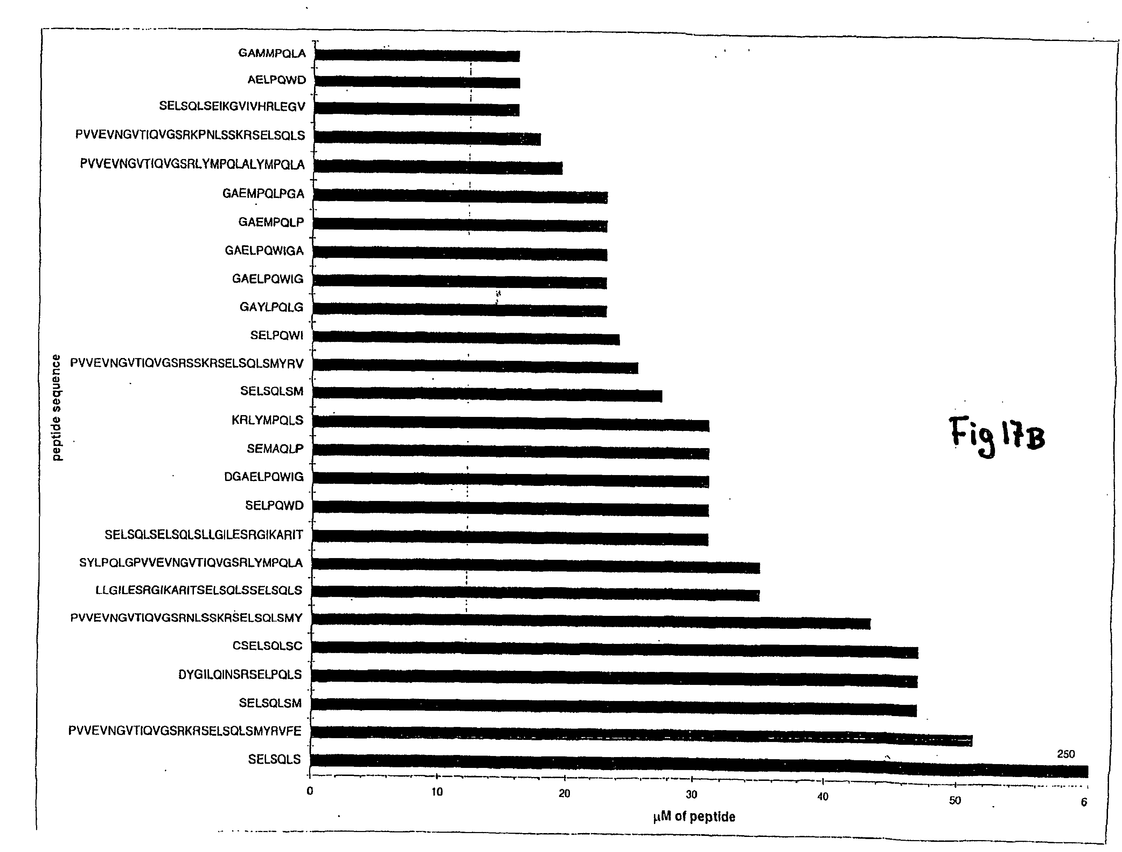

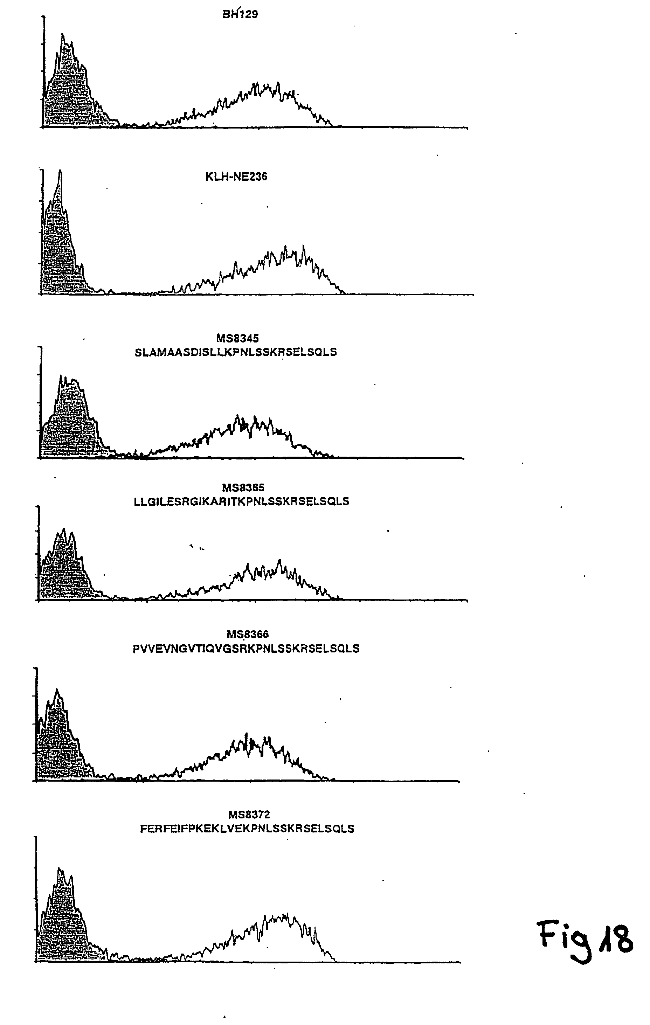

**Fig. 17** shows the inhibition of binding of saturating concentrations of BH129 to immobilized measles virus by mimotopes (competitor). Data are shown as 50% inhibiting concentrations (IC50%).

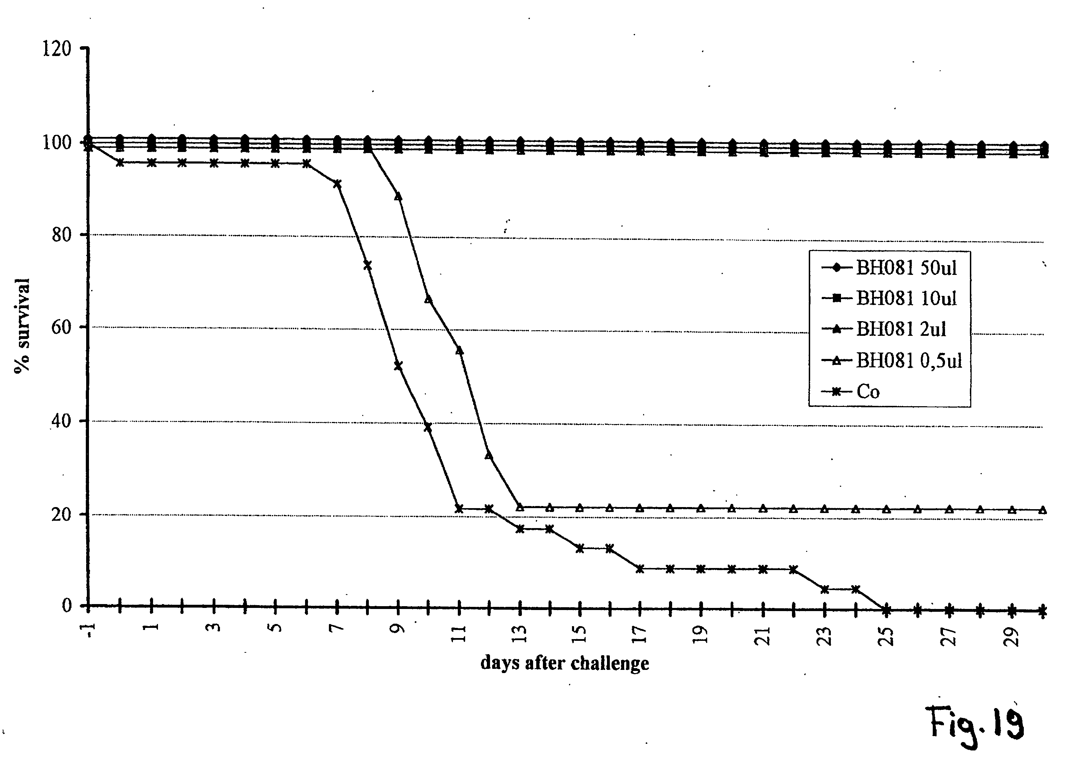

**Fig. 18.** shows the binding of serum of mice immunized with 50 µg of peptides (shown in each panel) mimicking (mimotopes) the binding site of BH129 to MV-. infected EBV-transformed human B cells (WMPT) measured by flow cytometry. Background binding corresponds to serum reactivity on non-infected cells. The data are expressed on a log-scale (3 decades) as arbitrary fluorescence units (AFU).
**Fig. 19.** shows a challenge/protection experiment with mice passively immunized on day -1 with 0.5-50 µg BH81. On day 0, mice were challenged with an intracranial injection of CAM mouse adapted MV. Naive mice were challenged without prior antibody transfer. Each group represents 5 to 10 mice from 2 independent experiments.

### Detailed Description of Preferred Embodiments

The term "antigenic determinant" as used herein, designates the structural component of a molecule that is responsible for specific interaction with corresponding antibody (immunoglobulin) molecules elicited by the same or related antigen.

The term "sequential antigenic determinant" as used herein designates an antigenic determinant wherein the structural components of a molecule that are responsible for specific interaction with corresponding antibody (immunoglobulin) molecules elicited by the same or related antigen, are contained within a sequence of 30 amino acids of a protein sequence. Sequential antigenic determinants in the present peptides comprise chemically active surface groupings of a sequence of less than 30 amino acid residues independent (i) of whether the antigenic determinant requires a specific conformation or not, and (ii) independent of the location of the structural components within the amino acid sequence of the viral protein. Antigenic determinants to which this ad hoc definition does not apply are here referred to as "conformation antigenic determinants".

The term "antigen" as used herein, means an entity that is bound by an antibody.

The term "immunogen" as used herein, describes an entity that induces antibody production in the host mammal. In some instances the antibody and the immunogen are the same entity while in other instances the two entities are different.

The phrase "immunologically mimics" is used herein to mean that an immunogenic peptide is not a natural protein or a cleaved fragment of a natural protein, but a manufactured polypeptide which induces production of antibodies that bind to the inducing polypeptide and also to the naturally occurring protein which it replaces. The peptide may have the same or a similar amino acid sequence or may have a completely different sequence which mimicks the peptide. The latter peptide is referred to as a mimotope.

The term "peptide" as used herein, describes an entity that is composed of a determined sequence of amino acid residues. "peptide" means that it can be produced by any well known methods of producing peptides, including the production by an organism which has eventually been genetically engineered, and then subsequently extracted, purified and/or modified.

All amino acids residues identified herein are in the natural or L-configuration unless otherwise specified. Standard single letter nomenclature has been used to designate amino acid residues.

### Description of Methods and Materials

### Reagents.

The MV Edmonston strain (ATCC VR-24 was grown in Vero cell cultures and its early passage supernatants were used for neutralization assays. For Western blot and for one immunization (BH216) the virus was further concentrated and purified by sucrose gradient centrifugation (Muller *et al*., J. Gen. Virol., 76, 1371-1380 (1995)). Virus used for all other immunizations was purified by affinity chromatography and inactivated by propiolactone. Ltk-cells transfected with H (Ltk-H; kindly provided by Drs. Wild and Beauverger; Beauverger *et al*., J. Virol. Methods, 44, 199-210 (1993), J. Gen. Virol., 74, 2357-2363 (1994)) and a persistently infected human EBV-transformed B cell line (WMPT) were used to test the reactivity of the monoclonal antibodies with MV or MV-H protein by flow cytometry as described (Muller et *al*., J. Gen. Virol., 76, 1371-1380 (1995)).

### Production of monoclonal antibodies.

8-10 week old Balb/c mice were immunized by intraperitoneal or multifocal subcutaneous injections with purified native or denatured (5 min. boiling in 1 mg/ml SDS under non-reducing conditions) MV emulsified in incomplete Freund's Adjuvant (Sigma, USA). The animals were boosted with the same antigen preparation on day 21 and 38 (denatured MV) or day 49 (native MV). On day 41 or 52, the spleen cells were explanted and fused with Sp2/0 myeloma cells to generate monoclonal antibodies. Hybridomas specific for the MV by ELISA or by Western blot were cloned by limiting dilution in Dulbecco's modified Eagle medium (DMEM) supplemented with 10 mM hypoxanthine, 1 mM glutamine, 1 mM sodium pyruvate, 100 IU/ml penicillin, 100 µg/ml streptomycin and 10% fetal bovine serum (FBS; all products from GIBCO). Antibodies were purified from the same but FBS-free medium or from ascites and purified by affinity chromatography on a protein G column (Pharmacia, Sweden). Concentrations were estimated by O.D. 280 nm. Ig subclasses and isotypes were determined with the mouse monoclonal antibody isotyping kit (Hycult Biotechnology, NL).

### Peptides.

121 pentadecapeptides overlapping by 10 amino acids and covering the whole sequence of the MV-H protein (Edmonston strain; Alkhatib & Briedis, Virology, 150, 479-490 (1986)) and truncation, elongation and substitution analogues of peptide H381 and H386 were synthesized by simultaneous multiple peptide synthesis (Wiesmüller *et al*., J. Gen. Virol., 73, 2211-2216 (1992)). Of course, the peptides may also be produced by any other well known method. The peptides were modified C-terminally by two ε-amino caproic acid residues and one lysine residue which separate the peptide from the biotin residue which was coupled to the Nε-amino group of lysine amide. In Fig. 4, peptides were biotinylated at their N-terminus using a similar spacer. All peptides are designated by the position of their N-terminal amino acid and correspond to 15mers unless stated otherwise Whenever stated, peptides were reduced with 140 mM 2-mercaptoethanol. Even much higher concentrations did not directly interfere with the ELISA assay, as was measured with an antibody specific for a peptide containing a single Cys. During the different ELISA steps reduced peptides were protected with 0.01% vitamin C from oxidation. When indicated, peptides were oxidized by bubbling air through a 5 µg/ml solution. Some peptides including truncation-, elongation- and glycin-substitution analogues have been synthesized as free peptides, which were used in competition peptide ELISA.

### ELISA.

MV-ELISA. MV-specific antibodies were detected using a commercial ELISA based on human diploid cells (Enzygnost, Behringwerke Marburg).For the titration of the mAbs, microtiter plates coated overnight with MV concentrate were used.

### Peptide ELISA.

The ELISA based on the biotinylated peptides was described earlier (Fournier *et al*., Int. Immunol. 1995 in press). In brief, microtiter plates (Maxisorp, NUNC, Denmark) were coated with 20 µg/ml highly purified streptavidin (a gift by Dr. L. Seik, Mediagnost, Tübingen, D). Biotinylated peptides (5 µg/ml) were immobilized by incubation for at least 1 hour at room temperature. Binding of the biotinylated peptides to the plate was verified by competition with biotinylated alkaline phosphatase (Vector Laboratories, USA). Free binding sites were saturated with 1% BSA (Sigma, USA) in TBS. In some assays with monoclonal antibodies the blocking buffer contained 10% BSA, 10% saccharose and 2% v/v normal goat serum (ICN Biomedical, Belgium) in PBS. The plates were either incubated for 1.5 hours at 37°C or overnight at 4°C with mouse sera or hybridoma supernatants diluted 1/200- or 1/20-fold respectively in dilution buffer (10 mM Tris buffered saline, 1% BSA, 0.1% Tween, pH 7.4) respectively. The ELISAs were developed with alkaline phosphatase-conjugated goat immunoglobulin (whole molecule) specific for γ-chain of mouse or human IgG (Sigma, St. Louis, USA) at a dilution of 1:500 in dilution buffer using p-nitrophenylphosphate (Sigma, USA) as a substrate. Absorbance was measured at 405 nm.

### Peptide competition ELISA.

Microtiter plates were coated with measles virus overnight and blocked with blocking buffer. Premixed solution of saturated concentration of monoclonal antibody and decreasing concentrations of peptide were added to the plate and incubated for one hour. The ELISA was developed as described above.

### Neutralization (NT), hemagglutination inhibition (HI) and plaque reduction (PR) assays.

NT and HI were performed as described earlier (Muller et al., J.Gen. Virol. 76:1371; 1995). The plaque reduction assay was done by preincubating 5-fold dilutions of purified mAbs with 40 pfu MV for 1h at 4°C in DMEM containing 2% Ultraser and antibiotics. Then the mixture was added to Vero cells grown in the same medium. After 1h incubation at 37°C, the inocculum was washed out. The cells were covered with the same tissue culture medium containing 1% agar gel. The number of plaques were counted after staining with neutral red.

### In vivo challenge/protection experiments.

13-16 day old, specific pathogen-free (SPF) Balb/c mice were treated with a single intraperitoneal injection of purified monoclonal antibodies (56-364 µg). 1 day later they were challenged by intracerebral injection of 12500 TCID₅₀ of Balb/c adapted CAM strain derived from the CAM/RB strain (a gift from Dr. Liebert, Würzburg, D; Liebert & ter Meulen, J. Gen. Virol., 68, 1715-1722 (1987)).

### Mouse sera.

Mice (CBA or Balb/c) were immunized with on day 0, and boosted on day 14 with 25ug peptide coupled to KLH-maleimide-Cys emulsified in CFA/IFA. Animals were bled on day 21.

### Method for selection of phages.

The screening was performed essentially according to the standard method described by Parmley and Smith, Gene 73:305-318 (1988). Panning tubes (Maxisorb, Nunc) were coated with 20 µg of antibody and after blocking with the above blocking buffer, they were incubated with 2x10¹¹ infective phages from the library. After washing off the non-binders, the bound phages were eluted from the tubes and amplified in bacteria. After three rounds of screening, a negative selection on an irrelevant antibody was done to select specific phages.

### Example 1

### Epitope mapping by MV-H peptides.

A panel of 60 H-specific clones were obtained after immunization with native or denatured MV. Linear epitopes of these clones were mapped using an ELISA based on biotinylated peptides. 7 monoclonal antibodies were found to react with peptides corresponding to the amino acids 361-410 . MAb BH168 and BH171 reacted with peptides H361/H366, BH6, BH21 and BH216 with H381/H386 (Fig. 1), BH147 and BH195 with H386/H391/H396. The specificity of the binding has been confirmed by competition with free amide peptides (data not shown).

### Functional activity of MV-H peptide-specific monoclonal antibodies.

The above monoclonal antibodies recognized the MV both in a certified diagnostic ELISA (Enzygnost, Behringwerke, Marburg) or when purified MV was coated to the plates (**Table 1**). BH6, BH21 and BH216 reacted about 500-times stronger than BH171, 168 or 195 (data not shown). Under non-reducing conditions they all identified a 160 kD protein in the Western blot, corresponding to the dimeric form of the H protein (Gerlier *et al*., J. Gen. Virol., 69, 2061-2069 (1988); Rima, J. Gen. Virol., 64, 1205-1219 (1983)). This was confirmed both with purified MV and recombinant H protein. Under reducing conditions, BH168, BH171, BH147 and BH195 reacted with an 80 kD protein, while the reactivity of BH6, BH21 and BH216 with the MV was lost. Monoclonal antibodies BH6, BH21 and BH216 recognized H on the surface of the MV-infected EBV-transformed cell line and the H-transfected Ltk cells. BH171, BH168, BH147 and BH195 were essentially negative on these cells. (**Fig. 2**). Recombinant H protein inhibited binding of BH21, BH6 and BH216 to peptide H386 demonstrating that the same antibodies react with both antigens. Only BH6, BH21 and BH216 neutralized MV in vitro and inhibited hemagglutination of monkey erythrocytes (**Table 1**). Although the binding sites of these peptides partially overlapped (amino acids 286-400) with those of BH147 and BH195 the latter did not display any functional activities.

### Challenge/protection experiments with monoclonal antibodies.

2 week old Balb/c mice received between 65 and 344 mg mAb one day before and four days after the intracranial injection of 1.25x10⁴ TCID₅₀ of CAM/Balb brain homogenate. **Fig. 3** shows that 62+/-6 µg BH6, BH21, BH216 were sufficient to protect animals from lethal challenge. In contrast, a 5-6 fold higher dose of mAb BH147 and BH195 (which recognize an overlapping region) or BH168 and BH171 did not protect in vivo.

### Role of the disulfide bond.

BH6, BH21 and BH216 recognized the MV only under non-reducing conditions suggesting that a disulfide bridge is required for binding. The 15mer peptide H381 has a Cys in position 381 and shares two additional Cys in position 386 and 394 with peptide H386. A 20mer peptide (381-400) corresponding to the sequence of peptide H381 and H386 inhibited binding of monoclonal antibodies BH6, BH21 and BH216 to these 15mers, but inhibition was lost when the three Cys were replaced by amino butyric acid (Abu; data not shown).

**Fig. 4a** shows that BH6, BH21 reacted about 4 times stronger with peptide H381 than with H386, while BH216 reacts equally well with H381 and H386. The three monoclonal antibodies recognized the oxidized peptides 4-64 times more efficiently than the reduced species. MAb BH195 reacts with peptide H386 and H391 irrespective of the disulfide bond.

**Fig. 4b** shows that the substitution of Cys386 by Abu decreases binding of monoclonal antibodies BH6 and BH21 to oxidized H386 almost 100-fold. Substitution of Cys394, either alone or together with Cys386 completely obliterates binding of these monoclonal antibodies. Binding of BH216 was considerably less sensitive to reduction and to Abu-substitution. Virtually no difference in binding is found between oxidized and reduced Abu-derivatives. The reaction of BH195 is independent of Abu-substitution or the oxidation state. The results showed that a cystin loop is required for optimal binding of the neutralizing antibodies BH6, BH21 but not of BH216. However, it also shows that some of the reactivity is to a linear part of the peptide. Therefore, peptide H386 was truncated from the C-terminus.

C-terminal truncation of peptide H386 showed that both in the reduced or oxidized state of the 14mer is required for maximal binding of mAb BH6, BH21 and BH216, both in the reduced and oxidized state (**Fig. 4c**). The antibodies did not react with the 10mer or shorter peptides even at the highest concentrations used (not shown). C-terminal elongation did not further enhance binding. Thus, in addition to the S-S loop the C-terminal linear tail contributes to the binding affinity.

### Characterization of anti-peptide sera.

Adult Balb/c mice were immunized with different peptides emulsified in FCA and boosted with the same peptides 2 weeks later. The reactivity of the sera with homologous peptides and with MV is shown in **Figure 5A** respectively. After immunization of mice with peptide H381 coupled to KLH-maleimide-Cys sera were obtained which reacted with MV-infected WMPT cells (Figure 5B) but not with non-infected cells. Figure 6 shows that such anti-peptide sera can protect susceptible CBA mice against a lethal challenge of a rodent-adapted measles virus.

### Sequence alignment.

The sequence H381-405 of different measles virus isolates is fully conserved in all vaccine strains (**Fig. 7**). Limited mutations have been found in recent wild-type isolates. Most mutations were found in the IP3 strain, an SSPE isolate from the early 1970s, which was shown to have a markedly reduced hemadsorption activity (Cattaneo *et al*., Virology, 173, 415-425 (1989); Burnstein et *al*., Infection and Immunity, 10, 1378-1382 (1974)). Escape mutants have been described in this region by Hu *et al*., Virology, 192, 351-354 (1993) and Liebert *et al*., J. Virol., 68, 1486-1493 (1994). The H protein of the MV is about 60% homologous with the RPV (Tsukiyama et al., Virology, 160, 48-54 (1987); Yamanaka et al., Virology, 166, 251-253 (1988)) and about 34% with CDV (Curran et al., J. Gen. Virol., 72, 443-447 (1991)). The amino acid sequence 381 to 400 of the H-protein of different morbillivirus (Fig. 7) shows very little homology but the Cys residues are fully conserved, indicating that these Cys are functionally critical.

### Discussion.

After immunization with native or denatured MV, several monoclonal antibodies were obtained, which reacted with peptides corresponding to the sequence H361-410 **(Table 1).** Three monoclonal antibodies (BH6, BH21, BH216) inhibited virus induced hemagglutination of monkey erythrocytes, neutralized the virus in vitro, and protected animals from a lethal challenge of rodent adapted neurotropic MV. These three monoclonal antibodies reacted with the 15mer peptides H381 and H386 defining H381-400 as a linear neutralizing and protective epitope. These peptides correspond to a small cysteine cluster region (Cys381, Cys386 and Cys394). In Western blot, BH6, BH21 and BH216 reacted with MV only under non-reducing conditions. For optimal binding of BH6 and BH21, but not necessarily for BH216 peptide H381 and H386 had to be oxidized, suggesting that an intramolecular disulfide bound which is formed by both peptides is sometimes required.

Studies with the Abu-analogues and the C-terminal truncations showed that there was also a low affinity binding component to linear parts of H386. Recognition of amino acids of the C-terminal tail (mainly (E)WA-COOH) by BH6, BH21 and BH216 does not require oxidation.

Two additional monoclonal antibodies, BH147 and BH195 mapped to the sequence H386-410 partially overlapping with the peptides recognized by the above neutralizing monoclonal antibodies. In contrast to the neutralizing antibodies, BH195 was insensitive to Abu-substitution and to reduction, demonstrating that these monoclonal antibodies did not require the loop structure for binding. C-terminal truncation analogues demonstrated that Trp399 was critical for binding of BH195.

The present antibodies against H381-400 region define a local loop protruding from the surface of the MV-H which contains an epitope involved in hemagglutination and neutralization ("HNE-loop") and which can be imitated by short peptide loops. Such peptides are of interest for being incorporated into a subunit vaccine. Proteins and peptides usually induce antibodies of the IgG1 subtype, which lacks the efficiency for complement fixation and FcγRI binding of the IgG2a isotype (Neuberger & Rajewsky, Eur.J.Immunol., 11, 1012-1016 (1981); Ravetch & Kinet, Annu.Rev.Immunol., 9, 457-492 (1991)), the most important effector antibody in viral infections (Ishizaka et al., The Journal of Infectious Diseases, 172, 1108-1111 (1995)). The present in vivo challenge experiments demonstrated that protection via monoclonal antibodies targeted to the HNE-loop is independent of these effector functions since both IgG1 and IgG2b isotypes protected.

Only limited numbers of mutations of the HNE-loop have been reported (**Fig. 7**) suggesting that this region does not tolerate extensive variability. Also the region bears homology with other morbilliviruses such as rinderpest and canine distemper virus and others. In particular, the conservation of the cysteines suggests that the HNE-domain is equally important in the other morbilliviruses and that our findings also extend to other morbilliviruses. It also indicates that the domain will not easily disappear under the selective pressure of antibodies.

The HNE region is defined by the following procedure:
the sequences of the morbillivirus will be aligned optimally with the Edmonston strain. Normally cysteines of the morbillivirus virus will align with the cysteines of cluster region defined by the cysteines in position 381, 386 and 394 of the Edmonston strain using standard procedures. If they do not fully align the nearest set of Cys with the corresponding intervals will be defined as those corresponding to 381, 386 and 394 of the Edmonston strain.

Following this procedure Cys381 will be designated Cys I, Cys 386 as Cys II, Cys394 as Cys III,

The functional region of the virus corresponds to the region from Cysl to Cys III plus at least 6 amino acids. A particular interesting sequence of the functional region is defined by the cysteine cluster region comprising at least two cysteines in position 386 and cysteine C394 of the Edmonston strain (C386KGKIQALC394ENPEWA)

Therefore, a peptide with at least 16 amino acids comprising at least two cysteine residues separated by between 4 and 14 other amino acids is capable of immunologically mimicking a morbillivirus in general and the measles virus in particular. The peptide has preferably less than 60 amino acids and most preferably less than 30 amino acids.

The two cysteine residues are separated preferably by a sequence of four (n = 4) or by seven (n = 7) other amino acid residues.

The peptide may comprise at least one third cysteine residue which is separated from one of the other cysteine residues by between 4 and 14 and preferably by 7 other amino acids.

The peptide is maybe in an oxidized or reduced form wherein said oxidized form has disulfide bridges between sulfur-containing amino acids.

One of the benefits of peptide-based MV vaccines is their potential to escape maternal antibodies transferred from the mother onto the child. Such a subunit vaccine could be used conceivably to induce neutralizing antibodies in the presence of antibodies from vaccinated or wild-type infected mothers

### Example 2

### MAb specificities.

A group of 9 mAbs reacted with peptides corresponding to amino acids 226-255 of the protein. BH1 recognized the pentadecamer (226-240) but not the free octamer (226-233). H226 in a competition ELISA, indicating that the amino acids 233-240 are critical for binding of this mAb. BH47, BH59, BH103 and BH129 were found to bind to peptides H236 and H241, covering the amino acids 236-255. The specificity of the binding has been confirmed by competition with free amide peptides and irrelevant peptides (data not shown). The mAbs generated a typical band of 80kD under denaturating and reducing conditions by Western blot. They reacted both with MV in a diagnostic ELISA (Enzygnost, Behringwerke, Marburg, D) and with MV purified from culture. The titration curves of the purified mAbs on MV and peptides is shown in **Figure 9**. The binding pattern of the mAbs BH59 and BH47 is very similar and differs from the other mAbs. The mAb BH129 differs from BH59 and BH47 in that it binds about 5-10 times more efficiently to the peptide H241 than to H236. All mAbs stained MV infected EBV cells (WMPPT, a gift from Dr. Chain, London), and with the exception of BH1 they also reacted with H transfected Ltk- cells (a kind gift from Dr. Wild Lyon, F) by flow cytometry (**Figure 10**). These experiments demonstrate that these antibodies bind the MV hemagglutinin protein and defined peptides derived from this protein.

### Fine mapping.

The epitope defined by the peptides H236 and H241 was further studied using free truncation analogues (**Figure 11**) and a series of peptides with monosubstitutions of each amino-acid by glycin (**Figure 12**). These data localized the epitope between amino acids 241-250. The amino acids E245, L246, Q248, and L249 were critical amino acids for binding of BH47, BH59, BH103 and BH129. For BH129 also S247 seems to play a role. The other amino acids did not seem to be substantially involved in the binding of these mAbs.

### Functional activities of mAbs.

Table 2 shows that BH47, BH59 and BH129 neutralize the measles virus in vitro. They have low or no hemagglutination inhibition activity.

### Challenge/protection experiments.

Purified BH47, BH59, BH103 and BH129 were injected intraperitoneally into 3 week old CBA mice, which were challenged two days later with a lethal dose of CBA-adapted MV. These mAbs gave full protection at a concentration of 375 µg/mouse whereas the mAbs BH189 and BH164 did not at twice this concentration (**Fig. 13**).

### Characterization of anti-peptide sera.

Adult Balb/c mice were immunized with peptide H236 (15mer) coupled to KLH-maleimide-Cys emulsified in FCA and boosted with the same conjugate 2 weeks later. Sera reacted with MV-infected WMPT cells (**Figure 14**) but not with non-infected cells. Such anti-peptide sera can protect susceptible CBA mice against a lethal challenge of a rodent-adapted measles virus (data not shown).

### Sequence alignment.

The amino acid sequence 241-250 of the MV-H showed a high degree of conservation (Fig. 14).

The binding site of several monoclonal antibodies which neutralized the measles 5 virus in vitro were characterized. Moreover, these mAbs protected against a lethal challenge of a mouse adapted MV. The antibodies were mapped to a linear neutralizing epitope corresponding to two 15-mer peptides (H236 and H241) sharing the amino acids SKRSELSQLS of the MV-H protein

### Sequences mimicking epitope 241-250 which are different from the virus sequence.

Phage displayed random 6-mer libraries were screened using standard methodology (Scott, J. K. and Smith, G. P., Science, 249 (1990) 386; Parmley, S.F. and Smith, G.P., Gene, 73 (1988) 305) with the monoclonal antibody BH129 specific for the sequential epitope of the measles virus hemagglutinin protein described in example 5 2. After two rounds of non-specific selection (panning on BH129) and one round of specific selection (panning on BH129 with blocked antigen binding sites or on an irrelevant isotype subclass-matched mAb) phages were obtained which shared only up to 3 amino acids with the wild-type epitope. While Gin is found back in 7 of 8 clones all competing phages contained a consensus Pro instead of the central Ser of the wild-type virus sequence. No clone with the wild-type sequence was selected and most clones contained a sequence motif not found in the wild-type sequence (LYMPQL, EMPQLP, ELPQWI, MMPQLA, FVPQWY, HYMFSV, YLPQLG). Mimotopes were designed and synthesized on the basis of these sequences and derivatives thereoff. The inhibition of the mAb binding to MV by these designer peptides was tested. The blocking capacity of most mimotopes was better than the wild-type sequence of the same length (7-mer; Fig 16). Figure 17 (A,B) represents the 50% inhibition concentration of a series of mimotopes of different length. The best mimotope blocked BH129 binding to the MV 40-120 better than the wild-type peptides. Fig.18 demonstrates that such mimotopes can also induce antibodies which recognize the measles virus.

### Example 3.

In example 2, mimotopes were developed against a sequential epitope. A phage displayed library with a random insert of the format CX9 as described by Koivunen et al. Biotechnology 13:265-270 was screened with monoclonal antibody BH81 using standard procedures described by Parmley and Smith, Gene 73:305-318, (1988). After non-selective and selective screening inserts were detected which specifically reacted with the binding site of BH81. BH81 is an example of an antibody which does not react with measles virus in the Western blot; which reacts with MV-infected WMPT cells but not uninfected WMPT cells; which reacts with MV-H transfected Ltk cells but wild-type Ltk cells; which does not react with the 15mer peptides corresponding to the hemagglutinin protein described by Ziegler et al. J. Gen. Virol. 77:2479-2489, 1996; BH81 inhibits hemagglutination. These data demonstrate that this antibody reacts with a conformational epitope of the MV-H protein. Furthermore, BH81 neutralizes the virus in vitro and protects in an animal model against a lethal challenge with rodent adapted measles virus (**Figure 19**).

The following peptides mimicking conformational epitopes of the MV-H protein were found (one letter code): 
(A) C D K V I R P A W C
(B) - - - D - Y - V - -
(C) - - R E - - L - Y -
 where line (A) represents the main sequence; lines (B) and (C) represent mutations which can occur as individual mutations or in different combinations. When these mimotopes were synthesized as peptides they inhibited binding of BH81 to the measles virus with an 50% inhibitory concentration of 0.1 to 50 µM. This demonstrates that linear peptides can also mimic neutralizing and protective conformational antigenic determinants of the measles virus hemagglutinin protein. Analogously as shown in example 1 and 2, derivatives of these peptides can also be used to induce MV-reactive antibodies in mammals.

## Claims

1. A vaccine comprising a peptide having an amino acid sequence corresponding to or mimicking at least one sequential antigenic determinant of the hemagglutinin protein of a morbillivirus, wherein said antigenic determinant is the binding site of a neutralizing and protective antibody of a morbillivirus.

2. The vaccine of claim 1 wherein the antigenic determinant is a binding site of an in vitro neutralizing and in vivo protective antibody of a morbillivirus.

3. The vaccine of any one of claims 1 to 2 wherein the protein is the hemagglutinin (H) protein of a measles virus (MV).

4. The vaccine of any one of claims 1 to 3 wherein said amino acid sequence comprises or mimicks at least a part of the amino acid sequence corresponding to amino acids 361 to 420 of the hemagglutinin protein of the Edmonston strain of MV or the corresponding domain of another morbillivirus, or a portion, variation, mutant or mimotope or conjugate thereof.

5. The vaccine of any one of claims 1 to 4 wherein said amino acid sequence comprises at least a part of the amino acid sequence corresponding to amino acids 381 to 400 of the hemagglutinin protein of the Edmonston strain of MV or the corresponding domain of another morbillivirus, or a portion, variation, mutant or mimotope or conjugate thereof.

6. The vaccine of any one of claims 1 to 5 wherein said amino acid sequence is selected from any of the amino acid sequences set forth in Fig. 7 or a portion, variation, mutant or mimotope or conjugate thereof.

7. The vaccine of any one of claims 1 to 6 wherein said peptide is in an oxidized or a reduced form wherein said oxidized form has disulfide bridges between sulfur-containing amino acids.

8. The vaccine of any one of claims 1 to 3 wherein said amino acid sequence comprises at least a part of the amino acid sequence corresponding to amino acids 226 to 255 of the hemagglutinin protein of the Edmonston strain of MV or the corresponding domain of another morbillivirus, or a portion, variation, mutant or mimotope or conjugate thereof.

9. The vaccine of claim 1 to 3 wherein said amino acid sequence comprises at least a part of the amino acid sequence corresponding to amino acids 241 to 250 of the hemagglutinin protein of the Edmonston strain of MV or the corresponding domain of another morbillivirus, or a portion, variation, mutant or mimotope or conjugate thereof.

10. The vaccine of any one of claims 3, 5, 8 and/or 9 wherein said amino acid sequence is selected from any of the amino acid sequences set forth in Fig. 15-18 or a portion, variation, mutant or mimotope or conjugate of any such sequence.

11. The vaccine of any of the preceding claims wherein the peptide described in any one the preceding claims has less than 60 amino acid residues and preferably less than 30 amino acid residues.

12. The vaccine of any one of claims 1 to 11 suitable for the prevention of morbilli virus infection.

13. The vaccine of claim 12 comprising the peptide in an amount of about 0.1 micrograms to about 100 milligrams.

14. The vaccine of claim 12 or 13 further comprising a pharmaceutically acceptable diluent, adjuvant, carrier, T cell epitope or excipient.

15. Use of the peptide described in any of the claims 1 to 11 for the manufacture of a medicament for the prevention and/or treatment of measles.

16. A sequential antigenic determinant comprising at least a part of the amino acid sequence corresponding to amino acids 361 to 420 of the hemagglutinin protein of the Edmonston strain of MV or a portion, variation, mutant or mimotope or conjugate thereof.

17. A sequential antigenic determinant comprising at least a part of the amino acid sequence corresponding to amino acids 226 to 255 of the hemagglutinin protein of the Edmonston strain of MV or a portion, variation, mutant or mimotope or conjugate thereof.

18. A peptide comprising at least one sequential antigenic determinant of claim 16 or 17.

## Patentansprüche

1. Impfstoff, welcher ein Peptid mit einer Aminosäuresequenz enthält, die mindestens einer der sequentiellen antigenen Determinanten des Hämagglutinin-Proteins eines Morbillivirus entspricht, oder diese imitiert, wobei die antigene Determinante die Bindestelle eines ein Morbillivirus neutralisierenden und davor schützenden Antikörpers darstellt.

2. Impfstoff nach Anspruch 1, wobei die antigene Determinante eine Bindestelle eines ein Morbillivirus in vitro neutralisierenden und in vivo davor schützenden Antikörpers darstellt.

3. Impfstoff nach einem der Ansprüche 1 bis 2, wobei das Protein das Hämagglutinin (H)-Protein eines Masernvirus (MV) ist.

4. Impfstoff nach einem der Ansprüche 1 bis 3, wobei die Aminosäuresequenz zumindest einen Teil der Aminosäuresequenz umfaßt oder imitiert, welche den Aminosäuren 361 bis 420 des Hämagglutinin-Proteins vom Edmonston-Stamm des Masernvirus, oder der entsprechenden Domäne eines anderen Morbillivirus, oder einem Teil, einer Variation, einer Mutante oder einem Mimotop oder einem Konjugat davon entspricht.

5. Impfstoff nach einem der Ansprüche 1 bis 4, wobei die Aminosäuresequenz zumindest einen Teil der Aminosäuresequenz umfaßt, welche den Aminosäuren 381 bis 400 des Hämagglutinin-Proteins vom Edmonston-Stamm des Masernvirus, oder der entsprechenden Domäne eines anderen Morbillivirus, oder einem Teil, einer Variation, einer Mutante oder einem Mimotop oder einem Konjugat davon entspricht.

6. Impfstoff nach einem der Ansprüche 1 bis 5, wobei die Aminosäuresequenz aus einer der Aminosäuresequenzen aus Fig. 7 oder einem Teil, einer Variation, einer Mutante oder einem Mimotop oder einem Konjugat davon ausgewählt ist.

7. Impfstoff nach einem der Ansprüche 1 bis 6, wobei das Peptid in einer oxidierten oder einer reduzierten Form vorliegt, und die oxidierte Form Disulfidbrücken zwischen Schwefel enthaltenden Aminosäuren hat.

8. Impfstoff nach einem der Ansprüche 1 bis 3, wobei die Aminosäuresequenz zumindest einen Teil der Aminosäuresequenz umfaßt, welche den Aminosäuren 226 bis 255 des Hämagglutinin-Proteins vom Edmonston-Stamm des Masernvirus, oder der entsprechenden Domäne eines anderen Morbillivirus, oder einem Teil, einer Variation, einer Mutante oder einem Mimotop oder einem Konjugat davon entspricht.

9. Impfstoff nach den Ansprüchen 1 bis 3, wobei die besagte Aminosäuresequenz zumindest einen Teil der Aminosäuresequenz umfaßt, welche den Aminosäuren 241 bis 250 des Hämagglutinin-Proteins vom Edmonston-Stamm des Masernvirus, oder der entsprechenden Domäne eines anderen Morbillivirus, oder einem Teil, einer Variation, einer Mutante oder einem Mimotop oder einem Konjugat davon entspricht.

10. Impfstoff nach einem der Ansprüche 3, 5, 8 und/oder 9, wobei die Aminosäuresequenz aus einer der Aminosäuresequenzen aus den Figuren 15-18 oder einem Teil, einer Variation, einer Mutante oder einem Mimotop oder einem Konjugat einer dieser Aminosäuresequenzen ausgewählt ist.

11. Impfstoff nach einem der vorhergehenden Ansprüche, wobei das Peptid, welches in einem der vorhergehenden Ansprüche beschrieben ist, weniger als 60 Aminosäuren und vorzugsweise weniger als 30 Aminosäuren besitzt.

12. Impfstoff nach einem der Ansprüche 1 bis 11, welcher zur Vorbeugung gegen eine Morbillivirus-Infektion geeignet ist.

13. Impfstoff nach Anspruch 12, welcher das Peptid in einer Menge von ungefähr 0,1 Mikrogramm bis ungefähr 100 Milligramm enthält.

14. Impfstoff nach Anspruch 12 oder 13, welcher außerdem ein Verdünnungsmittel, ein Adjuvans, einen Träger, ein T-Zell-Epitop oder einen Arzneistoffträger enthält, die pharmazeutisch verträglich sind.

15. Verwendung des Peptids, welches in einem der Ansprüche 1 bis 11 beschrieben ist, zur Herstellung eines Medikaments zur Vorbeugung und/oder zur Behandlung von Masern.

16. Sequentielle antigene Determinante, welche zumindest einen Teil der Aminosäuresequenz umfaßt, die den Aminosäuren 361 bis 420 des Hämagglutinin-Proteins vom Edmonston-Stamm des Masernvirus, oder einem Teil, einer Variation, einer Mutante oder einem Mimotop oder einem Konjugat davon entspricht.

17. Sequentielle antigene Determinante, welche zumindest einen Teil der Aminosäuresequenz umfaßt, die den Aminosäuren 226 bis 255 des Hämagglutinin Proteins vom Edmonston-Stamm des Masernvirus, oder einem Teil, einer Variation, einer Mutante oder einem Mimotop oder einem Konjugat davon entspricht.

18. Peptid, welches zumindest eine der sequentiellen antigenen Determinanten nach Anspruch 16 oder 17 enthält.

## Revendications

1. Un vaccin comprenant un peptide ayant une séquence en acides aminés correspondant ou mimant au moins un déterminant antigénique séquentiel de la protéine hémagglutinine d'un morbillivirus, ledit déterminant antigénique étant le site de fixation d'un anticorps neutralisant et protecteur dirigé contre un morbillivirus.

2. Le vaccin selon la revendication 1, dans lequel le déterminant antigénique est un site de fixation d'un anticorps, neutralisant in vitro et protecteur in vivo, dirigé contre un morbillivirus.

3. Le vaccin selon l'une quelconque des revendications 1 à 2, dans lequel la protéine est l'hémagglutinine (H), protéine d'un virus de la rougeole (MV).

4. Le vaccin selon l'une quelconque des revendications 1 à 3, dans lequel ladite séquence en acides aminés comprend ou mime au moins une partie de la séquence en acides aminés qui correspond aux acides aminés 361 à 420 de la protéine hémagglutinine du virus de la rougeole de la souche Edmonston, ou au domaine correspondant d'un autre morbillivirus ou à une portion, une variation, un mutant, un mimotope ou un conjugué de ceux-ci.

5. Le vaccin selon l'une quelconque des revendications 1 à 4, dans laquelle ladite séquence en acides aminés comprend au moins une partie de la séquence en acides aminés qui correspond aux acides aminés 381 à 400 de la protéine hémagglutinine du virus de la rougeole de la souche Edmonston, ou au domaine correspondant d'un autre morbillivirus, ou à une portion, une variation, un mutant ou un mimotope ou un conjugué de ceux-ci.

6. Le vaccin selon l'une quelconque des revendications 1 à 5, dans lequel ladite séquence en acides aminés est choisie parmi une des séquences en acides aminés présentées dans la figure 7, ou parmi une portion, une variation, un mutant ou un mimotope ou un conjugué de ceux-ci.

7. Le vaccin selon l'une quelconque des revendications 1 à 6, dans lequel ledit peptide est sous forme oxydée ou réduite, la forme oxydée possédant des ponts disulfures entre des acides aminés contenant du soufre.

8. Le vaccin selon l'une quelconque des revendications 1 à 3, dans lequel ladite séquence en acides aminés comprend au moins une partie de la séquence en acides aminés, qui correspond aux acides aminés 226 à 255 de la protéine hémagglutinine du virus de la rougeole de la souche Edmonston, ou au domaine correspondant d'un autre morbillivirus, ou à une portion, une variation, un mutant ou un mimotope ou un conjugué de ceux-ci.

9. Le vaccin selon les revendications 1 à 3, dans lequel la séquence en acides aminés comprend au moins une partie de la séquence en acides aminés, qui correspond aux acides aminés 241 à 250 de la protéine hémagglutinine du virus de la rougeole de la souche Edmonston, ou au domaine correspondant d'un autre morbillivirus ou à une portion, une variation, un mutant ou un mimotope ou un conjugué de ceux-ci.

10. Le vaccin selon l'une quelconque des revendications de 3, 5, 8 et/ou 9, dans lequel ladite séquence en acides aminés est sélectionnée parmi une quelconque des séquences en acides aminés présentées dans les figures 15 à 18, ou parmi une portion, une variation, un mutant ou un mimotope ou une conjugaison d'une de ses séquences.

11. Le vaccin selon l'une quelconque des revendications précédentes, dans lequel le peptide décrivé dans une quelconque des revendications précedentes a un nombre d'acides aminés inférieur à 60 et préférablement inférieur à 30.

12. Le vaccin selon l'une quelconque des revendications 1 à 11, approprié pour la prévention d'une infection causée par un Morbillivirus.

13. Le vaccin selon la revendication 12, contenant le peptide en quantité d'environ 0,1 microgrammes à environ 100 milligrammes.

14. Le vaccin selon la revendication 12 ou 13, contenant en plus un diluent, adjuvant, transporteur, épitope de cellule T ou excipient, pharmaceutiquement acceptables.

15. Utilisation d'un peptide décrit dans l'une quelconque des revendications 1 à 11 pour la fabrication d'un médicament pour la prévention et/ou le traitement de la rougeole.

16. Un déterminant antigénique séquentiel comprenant au moins une partie de la séquence en acides aminés, qui correspond aux acides aminés 361 à 420 de la protéine hémagglutinine du virus de la rougeole de la souche Edmonston ou à une portion, une variation, un mutant ou un mimotope ou un conjugué de ceux-ci.

17. Un déterminant antigénique séquentiel comprenant au moins une partie de la séquence en acides aminés, qui correspond aux acides aminés 226 à 255 de la protéine hémagglutinine du virus de la rougeole de la souche Edmonston ou à une portion, une variation, un mutant ou un mimotope ou un conjugué de ceux-ci.

18. Un peptide comprenant au moins un des déterminants antigéniques séquentiels des revendications 16 ou 17.
